# EUROPEAN PATENT SPECIFICATION

(11) **EP 3 779 457 B1**
(45) Date of publication and mention of the grant of the patent: **29.04.2026**
(21) Application number: 19776722.1
(22) Date of filing: 13.03.2019
(51) Int. Cl.: G01N 33/92, G01N 21/76, G01N 33/53, G01N 33/535, G01N 33/543

(54) **METHOD AND REAGENT FOR MEASURING UPTAKE PERFORMANCE OF LIPOPROTEIN**
VERFAHREN UND REAGENZ ZUR MESSUNG DER AUFNAHMELEISTUNG VON LIPOPROTEIN
PROCÉDÉ DE MESURE DE CAPACITÉ D'APPORT EN LIPOPROTÉINE, ET RÉACTIF

(30) Priority: 30.03.2018 JP 2018067824
(43) Date of publication of application: 17.02.2021
(73) Proprietor: SYSMEX CORPORATION, Kobe-shi Hyogo 651-0073 (JP)
(72) Inventor: HARADA Amane, Kobe-shi, Hyogo 651-0073 (JP); IINO Takuya, Kobe-shi, Hyogo 651-0073 (JP); KUBO Takuya, Kobe-shi, Hyogo 651-0073 (JP); MURAKAMI Katsuhiro, Kobe-shi, Hyogo 651-0073 (JP); SAIKI Naoya, Kobe-shi, Hyogo 651-0073 (JP); PENG Xiaoling, Kobe-shi, Hyogo 651-0073 (JP); KIRIYAMA Maria, Kobe-shi, Hyogo 651-0073 (JP)
(74) Representative: Hoffmann Eitle
(86) International application number: PCT/JP2019/010298
(87) International publication number: WO 2019/188297

(56) References cited:
- EP-A1- 3 225 993
- WO-A1-2012/011554
- WO-A1-2016/194825
- JP-A- 2004 533 236
- JP-A- 2012 530 900
- JP-A- 2016 080 685
- JP-A- H08 313 518

## Description

### TECHNICAL FIELD

The present invention relates to a method for measuring the uptake capacity of lipoproteins as defined in the appended claims.

### BACKGROUND ART

Lipid metabolic disorder is involved in various diseases, and the lipid concentration in blood is known to be an indicator of such diseases. However, in some cases, the lipid concentration does not reflect the presence of the diseases, the risk of the diseases, and the like. Therefore, a qualitative indicator focused on functions of lipoproteins is attracting attraction as well as a quantitative indicator such as the lipid concentration.

For example, even when the high-density lipoprotein cholesterol (HDL-C) concentration in blood is high, the risk of cardiovascular disease (CVD) is not reduced in some cases. Therefore, a possibility that the HDL-C concentration does not completely reflect the risk of CVD has been pointed out. Functions of high-density lipoprotein (HDL) have been focused on, and it is reported that excretion of cholesterol from peripheral tissues by HDL is a negative prognostic factor for the risk of CVD.

As a method for studying the functions of HDL, for example, PATENT LITERATURE 1 describes a method for measuring the cholesterol uptake capacity of lipoproteins. The method includes: a step of mixing a lipoprotein in a sample, a cholesterol added with a tag, and an antibody that specifically binds to the lipoprotein, thereby forming a complex that includes the antibody and the lipoprotein incorporating the cholesterol added with the tag; a step of labeling the complex by causing the complex, a capture body specifically binding to the tag, and a labeling substance, to be bound to one another; and a step of detecting a signal generated by the labeling substance bound to the complex.
EP 3 225 993 A1 describes a method for measuring the cholesterol uptake capacity of lipoproteins.

### CITATION LIST

### [PATENT LITERATURE]

[PTL 1] US Patent Application Publication No. 2017/0315112

### SUMMARY OF THE INVENTION

### PROBLEMS TO BE SOLVED BY THE INVENTION

In a measurement system for detecting a target substance, various nonspecific reactions and particle aggregation could occur. An object of the present invention is to suppress nonspecific reactions and particle aggregation in the measurement system.

### SOLUTION TO THE PROBLEMS

The present invention provides a method for measuring an uptake capacity of a lipoprotein as defined in the appended claims. The method includes the steps of: preparing a lipoprotein incorporating a labeled sterol, by mixing the lipoprotein in a sample and the labeled sterol in the presence of a surfactant having no cyclic structure; and measuring an uptake capacity of the labeled sterol on the basis of a label of the labeled sterol incorporated into the lipoprotein as defined in the appended claims.

Also disclosed isa method for measuring an uptake capacity of a lipoprotein. The method includes the steps of: preparing a lipoprotein incorporating a labeled sterol, by mixing the lipoprotein in a sample, the labeled sterol, and a surfactant having no cyclic structure; and measuring an uptake capacity of the labeled sterol on the basis of a label of the labeled sterol incorporated into the lipoprotein.

Also disclosed is a reagent for measuring an uptake capacity of a lipoprotein. The reagent comprises a labeled sterol and a surfactant having no cyclic structure.

Also disclosed is a sample diluting reagent for use in measurement of an uptake capacity of a lipoprotein. The sample diluting reagent comprises a surfactant having no cyclic structure.

Also disclosed is a washing reagent for use in measurement of an uptake capacity of a lipoprotein. The washing reagent comprises a surfactant having no cyclic structure.

### ADVANTAGEOUS EFFECTS OF THE INVENTION

According to the present invention, in measurement of the uptake capacity of a lipoprotein, nonspecific reactions and particle aggregation are suppressed.

### BRIEF DESCRIPTION OF THE DRAWINGS

[FIG. 1A] FIG. 1A shows an example of an external view of a reagent kit according to the present embodiment.
[FIG. 1B] FIG. 1B shows an example of an external view of a reagent kit according to the present embodiment.
[FIG. 1C] FIG. 1C shows an example of an external view of a reagent kit according to the present embodiment.
[FIG. 1D] FIG. 1D shows an example of an external view of a reagent kit according to the present embodiment.
[FIG. 2] FIG. 2 is a graph showing collection efficiency of magnetic particles.
[FIG. 3] FIG. 3 is a graph showing results of measurement of the cholesterol uptake capacity of HDL by using reaction buffers containing various surfactants.
[FIG. 4] FIG. 4 is a graph showing results of measurement of the cholesterol uptake capacity of HDL by using reaction buffers containing various surfactants.
[FIG. 5] FIG. 5 is a graph showing results of measurement of the cholesterol uptake capacity of HDL by using reaction buffers containing surfactants having no cyclic structure.
[FIG. 6] FIG. 6 is a graph showing results of measurement of the cholesterol uptake capacity of HDL by using reaction buffers containing surfactants having no cyclic structure.
[FIG. 7] FIG. 7 is a graph showing results of measurement of the cholesterol uptake capacity of HDL by using sample diluting reagents containing various surfactants.
[FIG. 8] FIG. 8 is a graph showing results of measurement of the cholesterol uptake capacity of HDL by using sample diluting reagents containing surfactants having no cyclic structure.
[FIG. 9] FIG. 9 is a graph showing results of measurement of the cholesterol uptake capacity of HDL by using sample diluting reagents containing surfactants having no cyclic structure and cyclodextrin.

### DESCRIPTION OF EMBODIMENTS

### [1. Method 1 for measuring uptake capacity of lipoprotein]

In a method for measuring the uptake capacity of a lipoprotein of the present embodiment (hereinafter, also referred to as "measurement method"), first, a lipoprotein in a sample and a labeled sterol are mixed in the presence of a surfactant having no cyclic structure. Through this mixing, the lipoprotein and the labeled sterol come into contact with each other in the presence of the surfactant having no cyclic structure, and the labeled sterol is incorporated into the lipoprotein.

In the present embodiment, as shown in Examples described later, the above-described mixing is performed in the presence of a surfactant having no cyclic structure, and the uptake reaction by the lipoprotein is promoted when compared with a case where a surfactant having a cyclic structure is used. Therefore, in the measurement method of the present embodiment, the uptake capacity of the lipoprotein can be measured with high accuracy. In addition, the surfactant having no cyclic structure also serves as a blocking agent. Therefore, in the present embodiment, it is not necessary to use an animal-derived protein, such as bovine serum albumin (BSA), that is generally used as a blocking agent in immunological measurement. As for the animal-derived protein, qualities between lots are not stable in some cases. In addition, when compared with a surfactant, the animal-derived protein tends to nonspecifically bind to a component in a sample, an antibody added to the measurement system, and a labeled sterol. Since the surfactant having no cyclic structure is a synthesized product, the quality is substantially constant, and influence on the measurement is very little when compared with the animal-derived protein.

### (Surfactant having no cyclic structure)

The surfactant having no cyclic structure denotes a surfactant that is an acyclic compound. The surfactant having no cyclic structure is a nonionic surfactant.

The nonionic surfactant having no cyclic structure is a polyalkylene glycol ethylene oxide adduct, and/or polyoxyethylene lauryl ether, The nonionic surfactant having no cyclic structure may be used individually or in combination of two or more.

In the present embodiment, polyoxyethylene lauryl ether is particularly preferable. Polyoxyethylene lauryl ether is commercially available. Examples thereof include NONION K-230 and NONION K-220 of NOF Corporation, EMULGEN 123P and EMULGEN 130K of Kao Corporation, and the like.

In the present embodiment, as the polyalkylene glycol ethylene oxide adduct, a block copolymer is preferable, and a triblock copolymer of ethylene oxide and propylene oxide is more preferable. As the nonionic surfactant comprising the block copolymer, a compound represented by formula (I) below is particularly preferable. 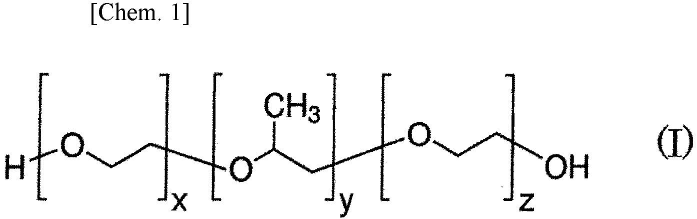 (where, x, y, and z are identical or different from each other, and are each an integer not smaller than 1, and
the molecular weight of the polypropylene oxide moiety is not greater than 2750)

The compound represented by formula (I) is also referred to as a polypropylene glycol ethylene oxide adduct, polyoxyethylene polyoxypropylene glycol, a polyoxyethylene polyoxypropylene block polymer, or a Pluronic (trademark)-type nonionic surfactant.

In formula (I), the molecular weight of the polypropylene oxide moiety (hereinafter, also referred to as "PPO") denotes a molecular weight of the moiety represented by -(O-CH(CH₃)-CH₂)y-, and is a molecular weight calculated from the molecular structural formula. In the present embodiment, the molecular weight of PPO is preferably not less than 900 and not greater than 2750, more preferably not less than 950 and not greater than 2750, and particularly preferably not less than 950 and not greater than 2250. PPO in the Pluronic-type nonionic surfactant has relatively high hydrophobicity. Thus, the greater the molecular weight of PPO, the greater the hydrophobicity of the surfactant. Therefore, a Pluronic-type nonionic surfactant that has a molecular weight of PPO greater than 2750 could act on the lipoprotein and inhibit uptake of the labeled sterol.

The average molecular weight of the compound represented by formula (I) is preferably 1000 to 13000. In formula (I), the sum of x and z is preferably 2 to 240. In formula (I), y is preferably 15 to 47, more preferably 16 to 47, and particularly preferably 16 to 39. Herein, the "average molecular weight" is a weight-average molecular weight measured by gel permeation chromatography (GPC).

The Pluronic-type nonionic surfactant is commercially available. Examples thereof include the Pluronic series of BASF, the PLONON series of NOF Corporation, the Adeka Pluronic series of Asahi Denka Co., Ltd., and the like. The Pluronic series is classified according to a Pluronic grid in which the vertical axis represents the molecular weight of PPO, and the horizontal axis represents the ethylene oxide (EO) content (%) (for example, see Pitto-Barry A. and Barry N.P.E., Poly. Chem., 2014, vol.5, p.3291-3297). The Pluronic grid is provided not only from academic literature but also from various manufacturers. In the present embodiment, the compound represented by formula (I) may be selected on the basis of the Pluronic grid.

Examples of the Pluronic-type nonionic surfactant represented by formula (I) include: Pluronic L31 (molecular weight of PPO: 950, EO content: 10%), Pluronic L35 (molecular weight of PPO: 950, EO content: 50%), Pluronic F38 (molecular weight of PPO: 950, EO content: 80%), Pluronic L42 (molecular weight of PPO: 1200, EO content: 20%), Pluronic L43 (molecular weight of PPO: 1200, EO content: 30%), Pluronic L44 (molecular weight of PPO: 1200, EO content: 40%), Pluronic L61 (molecular weight of PPO: 1750, EO content: 10%), Pluronic L62 (molecular weight of PPO: 1750, EO content: 20%), Pluronic L63 (molecular weight of PPO: 1750, EO content: 30%), Pluronic L64 (molecular weight of PPO: 1750, EO content: 40%), Pluronic P65 (molecular weight of PPO: 1750, EO content: 50%), Pluronic F68 (molecular weight of PPO: 1750, EO content: 80%), Pluronic L72 (molecular weight of PPO: 2050, EO content: 20%), Pluronic P75 (molecular weight of PPO: 2050, EO content: 50%), Pluronic F77 (molecular weight of PPO: 2050, EO content: 70%), Pluronic L81 (molecular weight of PPO: 2250, EO content: 10%), Pluronic P84 (molecular weight of PPO: 2250, EO content: 40%), Pluronic P85 (molecular weight of PPO: 2250, EO content: 50%), Pluronic F87 (molecular weight of PPO: 2250, EO content: 70%), Pluronic F88 (molecular weight of PPO: 2250, EO content: 80%), Pluronic L92 (molecular weight of PPO: 2750, EO content: 20%), Pluronic P94 (molecular weight of PPO: 2750, EO content: 40%), Pluronic F98 (molecular weight of PPO: 2750, EO content: 80%), and the like.

The triblock copolymer of ethylene oxide and propylene oxide may be a compound represented by formula (II) below. This compound is also referred to as a reverse Pluronic-type nonionic surfactant. 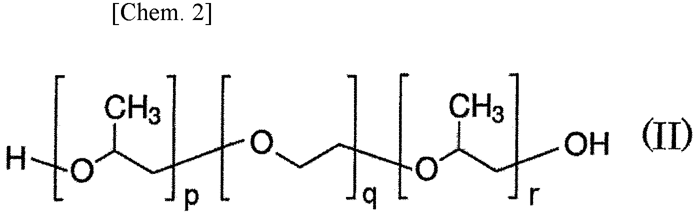 (where p, q, and r are identical or different from each other, and are each an integer not smaller than 1, and
the molecular weight of the polypropylene oxide moiety is not greater than 2750)

In formula (II), the molecular weight of the polypropylene oxide moiety denotes the total molecular weight of the moiety represented by -(O-CH(CH₃)-CH₂)ₚ- and -(O-CH(CH₃)-CH₂)ᵣ-, and is a molecular weight calculated from the molecular structural formula. In the present embodiment, the molecular weight of PPO is preferably not less than 900 and not greater than 2750, and more preferably not less than 950 and not greater than 2250. The Pluronic-type nonionic surfactant represented by formula (II) is commercially available. Examples thereof include Pluronic 10R5 (molecular weight of PPO: 950, EO content: 50%) of BASF, and the like.

The average molecular weight of the compound represented by formula (II) is preferably 1000 to 13000. In formula (II), q is preferably 2 to 240. In formula (II), the sum of p and r is preferably 15 to 47, more preferably 16 to 47, and particularly preferably 16 to 39. Herein, the "average molecular weight" is a weight-average molecular weight measured by GPC.

### (Sample that contains lipoprotein)

In the present embodiment, the sample is not particularly limited as long as the sample contains a lipoprotein of a mammal, preferably a lipoprotein of a human. Examples of the sample include a blood sample such as blood, serum, or plasma, and a sample obtained by diluting these.

In the present embodiment, the lipoprotein may be HDL, low-density lipoprotein (LDL), intermediate-density lipoprotein (IDL), very-low-density lipoprotein (VLDL), or chylomicron (CM). HDL is a lipoprotein having a density of not less than 1.063 g/mL. LDL is a lipoprotein having a density of not less than 1.019 g/mL and less than 1.063 g/mL. IDL is a lipoprotein having a density of not less than 1.006 g/mL and less than 1.019 g/mL. VLDL is a lipoprotein having a density of not less than 0.95 g/mL and less than 1.006 g/mL. CM is a lipoprotein having a density of less than 0.95 g/mL. Among these, the measurement method of the present embodiment is suitable for measurement of the uptake capacity of HDL.

In the present embodiment, a blood sample may be separated by a known method such as ultracentrifugation or a polyethylene glycol (PEG) precipitation method to obtain a fraction containing a predetermined lipoprotein. The fraction containing the predetermined lipoprotein obtained in this manner may be used as a sample.

Lipoproteins are known to esterify and incorporate cholesterol. When a labeled cholesterol described later is used as a labeled sterol, a fatty acid required for esterification reaction of cholesterol by the lipoprotein or a composition (for example, liposome) containing the fatty acid may be added to the sample.

In the present embodiment, the sample containing the lipoprotein may be diluted. For example, in order to adjust the lipoprotein concentration, a liquid obtained by diluting, with an aqueous medium, the above-described blood sample or fraction containing the predetermined lipoprotein can be used as a sample. Examples of the aqueous medium include water, saline, buffers such as phosphate buffered saline (PBS), Tris-HCl, and the like.

In the present embodiment, the sample containing the lipoprotein may be diluted in the presence of a surfactant having no cyclic structure. For example, the sample containing the lipoprotein may be diluted with a solution in which the surfactant having no cyclic structure is dissolved in the above-described aqueous medium. When the sample diluted in this manner and the labeled sterol are mixed together, the lipoprotein and the labeled sterol come into contact with each other in the presence of the surfactant having no cyclic structure.

The concentration of ApoAI, which is a major component of the lipoprotein, serves as an indicator of the concentration of the lipoprotein in the sample. In the present embodiment, a part of the sample is taken and the concentration of ApoAI contained therein may be measured by a known immunoassay (for example, immunonephelometry). The concentration of the lipoprotein in the sample can be adjusted by diluting the sample on the basis of the obtained ApoAI concentration.

In the present embodiment, the sample containing the lipoprotein may be diluted in the presence of a cyclic oligosaccharide. For example, the sample containing the lipoprotein may be diluted with a solution in which a cyclic oligosaccharide is dissolved in the above-described aqueous medium. Examples of the cyclic oligosaccharide include cyclodextrin, methyl-β-cyclodextrin, hydroxypropyl cyclodextrin, and the like. In the measurement method of the present embodiment, the cyclic oligosaccharide functions as a blocking agent by including the cholesterol derived from the sample.

In the present embodiment, the sample containing the lipoprotein may be diluted in the presence of a component that binds to a lipoprotein different from the lipoprotein to be measured. For example, the sample containing the lipoprotein may be diluted with a solution in which the component is dissolved in the above-described aqueous medium. Examples of the component that binds to a lipoprotein different from the lipoprotein to be measured include calixarene and the like. Calixarene binds to LDL, VLDL, and CM, but does not bind to HDL. When the uptake capacity by HDL is to be measured, if calixarene is added to the sample, LDL, VLDL, and CM derived from the sample can be masked.

In the present embodiment, the sample containing the lipoprotein may be diluted in the presence of a blocking agent for preventing nonspecific adsorption of the labeled sterol. For example, the sample containing the lipoprotein may be diluted with a solution in which the blocking agent is dissolved in the above-described aqueous medium. The addition of the blocking agent suppresses, for example, nonspecific adsorption of the labeled sterol to a capture body or a solid phase described later. The blocking agent may be any component that can suppress adsorption of the labeled sterol to a substance other than the labeled cholesterol. Examples of a preferable blocking agent include a 2-methacryloyloxyethyl phosphoryl choline-based polymer. This polymer may be a homopolymer of 2-methacryloyloxyethyl phosphoryl choline or a copolymer with another monomer. As another monomer, an acrylic ester or methacrylic acid ester having an alkyl group having 1 to 20 carbon atoms as a hydrophobic group is preferable. The 2-methacryloyloxyethyl phosphoryl choline-based polymer is commercially available. Examples thereof include the LIPIDURE (trademark) series of NOF Corporation. Among these, LIPIDURE-BL203 is particularly preferable.

### (Labeled sterol)

The labeled sterol is a sterol that has a labeling substance. Hereinafter, the labeling substance of the labeled sterol is also referred as a "first label". The sterol used in the labeled sterol is not particularly limited as long as the sterol is incorporated into the lipoprotein. Examples of the sterol include cholesterol and derivatives thereof. Examples of the cholesterol derivatives include precursors of bile acids, precursors of steroids, and the like. Specifically, 3β-hydroxy-Δ5-cholenoic acid, 24-amino-5-colen-3β-ol, and the like are preferable.

In the present embodiment, the labeled sterol is preferably cholesterol that has a labeling substance (hereinafter, also referred to as "labeled cholesterol"). The cholesterol moiety in the labeled cholesterol may have a structure of naturally occurring cholesterol, or a structure of cholesterol (also referred to as norcholesterol) obtained by removing not less than one methylene group and/or methyl group from an alkyl chain bound to C17 position of naturally occurring cholesterol.

Since lipoproteins incorporate cholesterol through esterification, it is preferable to use the labeled cholesterol that is esterified by lipoproteins. In the present embodiment, when the labeled cholesterol comes into contact with the sample, the labeled cholesterol is esterified by lecithin-cholesterol acyltransferase (LCAT) of a biological origin contained in the sample. Methods for confirming esterification of the labeled cholesterol by lipoproteins are known in the art and can be routinely performed by a person skilled in the art.

The first label is not particularly limited as long as the first label is a labeling substance that enables detection of the labeled sterol incorporated into the lipoprotein. The first label may be, for example, a tag that is itself to be detected, or may be a substance that generates a detectable signal (hereinafter, also referred to as a "signal generating substance").

The tag as the first label is not particularly limited as long as: the tag does not inhibit uptake of the sterol by the lipoprotein; and a substance capable of specifically binding to the tag is present or is obtained. Hereinafter, the sterol added with a tag as the first label is also referred to as a "tagged sterol". Similarly, cholesterol added with a tag as the first label is also referred to as a "tagged cholesterol". The tagged cholesterol is known in this technical field, and is described, for example, in US 2017/0315112 A1 (US 2017/0315112 A1 is incorporated herein by reference).

Examples of the tagged cholesterol include a tagged cholesterol represented by formula (III) below. 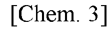 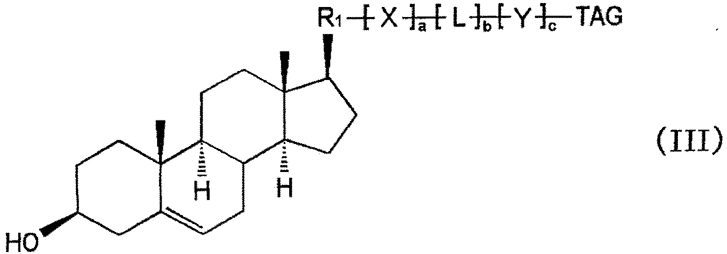 (where R₁ is an alkylene group having 1 to 6 carbon atoms and optionally having a methyl group,
X and Y are identical or different from each other, and are each represented by - R₂-NH-, -NH-R₂-, -R₂-(C=O)-NH-, -(C=O)-NH-R₂-, -R₂-NH-(C=O)-, -NH-(C=O)-R₂-, -R₂-(C=O)-, -(C=O)-R₂-, -R₂-(C=O)-O-, -(C=O)-O-R₂-, -R₂-O-(C=O)-, -O-(C=O)-R₂-, -R₂-(C=S)-NH-, -(C=S)-NH-R₂-, -R₂-NH-(C=S)-, -NH-(C=S)-R₂-, -R₂-O-, -O-R₂-, -R₂-S-, or -S-R₂-, and each R₂ is independently: an atomic bonding; an alkylene group having 1 to 10 carbon atoms and optionally having a substituent; an arylene group or heteroarylene group having 6 to 12 carbon atoms and optionally having a substituent; or a cycloalkylene group or heterocycloalkylene group having 3 to 8 carbon atoms and optionally having a substituent,
L is represented by -(CH₂)_{d}-[R₃-(CH2)ₑ]_{f}- or -[(CH₂)ₑ-R₃]_{f}-(CH₂)_{d}-, and R₃ is an oxygen atom, a sulfur atom, -NH-, -NH-(C=O)-, or -(C=O)-NH-,
TAG is a tag,
a and c are identical or different from each other, and are each an integer of 0 to 6,
b is 0 or 1,
d and e are identical or different from each other, and are each an integer of 0 to 12, and
f is an integer of 0 to 24.)

In formula (III), when a, b, and c are all 0, the tagged cholesterol represented by this formula has no linker, and the tag and the cholesterol moiety are directly bound to each other. In formula (III), when any one of a, b, and c is not 0, the tagged cholesterol represented by this formula has a linker (-[X]ₐ-[L]_{b}-[Y]_{c}-) between the tag and the cholesterol moiety. It is considered that the linker further facilitates the bonding between the tag exposed on the outer surface of the lipoprotein and the capture body. Each substituent of formula (III) is described below.

R₁ may have an alkylene group having 1 to 6 carbon atoms as a main chain, and may have a methyl group at any position. R₁ corresponds to an alkyl chain bound to the C17 position of naturally occurring cholesterol. In the present embodiment, when R₁ has 1 to 5 carbon atoms, R₁ preferably has a methyl group at the C20 position of the naturally occurring cholesterol. When R₁ has 6 carbon atoms, R₁ preferably has the same structure as that of alkyl chains at the C20 position to C27 position of the naturally occurring cholesterol.

[X]ₐ corresponds to a connecting moiety between R₁ and L, [Y]_{c}, or the tag. [Y]_{c} corresponds to a connecting moiety between R₁, [X]ₐ, or L, and the tag. X and Y are determined in accordance with the type of reaction in which the cholesterol moiety and the linker are bound to each other and reaction in which the linker and the tag are bound to each other.

With respect to R₂, the atomic bonding means a direct bonding without another atom intervening. When R₂ is an alkylene group having 1 to 10 carbon atoms, examples of the alkylene group include methylene, ethylene, propylene, isopropylene, butylene, isobutylene, pentylene, neopentylene, hexylene, heptylene, octylene, 2-ethylhexylene, nonylene, and decylene groups. Among these, an alkylene group having 1 to 4 carbon atoms is preferable. When R₂ is an alkylene group having a substituent, the above number of carbon atoms does not include the number of carbon atoms in the substituent.

When R₂ is an arylene group or a heteroarylene group, the group only needs to be an aromatic ring having 6 to 12 carbon atoms and optionally including not less than one hetero atom selected from N, S, O, and P. Examples of the group include phenylene, naphthylene, biphenylylene, furanylene, pyrrolene, thiophenylene, triazolene, oxadiazolene, pyridylene, and pyrimidylene groups. When R₂ is an arylene group or a heteroarylene group having a substituent, the above number of carbon atoms does not include the number of carbon atoms in the substituent.

When R₂ is a cycloalkylene group or a heterocycloalkylene group, the group only needs to be a non-aromatic ring having 3 to 8 carbon atoms and optionally including not less than one hetero atom selected from N, S, O, and P. Examples of the group include cyclopropylene, cyclobutylene, cyclopentylene, cyclohexylene, cycloheptylene, cyclooctylene, pyrrolidinylene, piperidinylene, and morpholinylene groups. When R₂ is a cycloalkylene group or heterocycloalkylene group having a substituent, the above number of carbon atoms does not include the number of carbon atoms in the substituent.

Examples of the substituent in R₂ include hydroxyl, cyano, alkoxy, =O, =S, -NO₂, -SH, halogen, haloalkyl, heteroalkyl, carboxyalkyl, amine, amide, and thioether groups. R₂ optionally has a plurality of the substituents. Here, halogen represents fluorine, chlorine, bromine, or iodine. Alkoxy represents an -O-alkyl group, and the alkyl group is a linear or branched saturated aliphatic hydrocarbon group having 1 to 5 carbon atoms, and preferably 1 or 2 carbon atoms.

Preferably, a and c are both 1, and X and Y are identical or different from each other and are each -(C=O)-NH- or -NH-(C=O)-.

L corresponds to a spacer and has a polymer structure that provides a predetermined length to the linker. It is preferable that the polymer structural moiety does not inhibit the uptake of cholesterol by the lipoprotein, and that the linker moiety is hardly incorporated into the lipoprotein. Examples of the polymer include a hydrophilic polymer such as polyethylene glycol (PEG). In a preferred embodiment, L is a structure represented by - (CH₂)_{d}-[O-(CH₂)ₑ]_{f}- or -[(CH₂)ₑ-O]_{f}-(CH₂)_{d}-. d and e are identical or different from each other, and are each an integer of 0 to 12, preferably an integer of 2 to 6, and more preferably both 2. f is an integer of 0 to 24, preferably an integer of 2 to 11, and more preferably an integer of 4 to 11.

The tag may be either a naturally occurring substance or a synthesized substance, and examples thereof include compounds, peptides, proteins, nucleic acids, and combinations thereof. The compound may be any labeling compound known in the art as long as a substance capable of specifically binding to the compound is present or obtained, and examples thereof include dye compounds and the like.

In this technical field, it is known that cholesterol is esterified to increase lipid solubility, thereby promoting uptake of cholesterol by lipoproteins. The tag attached to cholesterol may be a lipid soluble or hydrophobic substance.

Examples of combinations of a tag and a substance capable of specifically binding to the tag include: an antigen and an antibody that recognizes the antigen; a hapten and an anti-hapten antibody; a peptide or a protein and an aptamer that recognizes them; a ligand and a receptor thereof; an oligonucleotide and an oligonucleotide having a complementary strand thereof; biotin and avidin (or streptavidin); a histidine tag (a peptide containing histidine of 6 to 10 residues) and nickel; glutathione-S-transferase (GST) and glutathione; and the like. The antigen as a tag may be a peptide tag and a protein tag known in the art, and examples thereof include FLAG (registered trademark), hemagglutinin (HA), Myc protein, green fluorescent protein (GFP), and the like. Examples of the hapten as a tag include 2,4-dinitrophenol and the like.

Examples of the tagged sterol include sterols added with a structure represented by formula (IV) below: 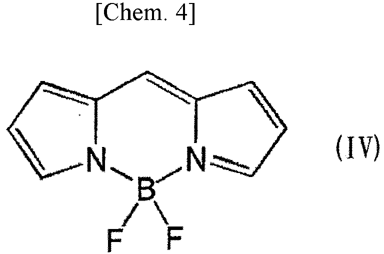 or a structure represented by formula (V) below: 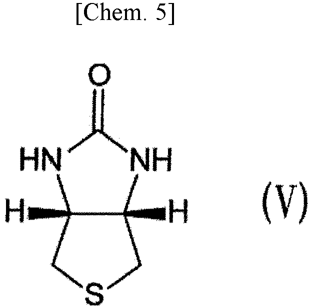 , as a tag. The structure represented by formula (IV) is a boron dipyrromethene (BODIPY (registered trademark)) backbone, and the structure represented by formula (V) shows a part of biotin. A sterol added with the structure represented by formula (IV) or (V) as a tag is preferable because capture bodies for these tags are generally available. A sterol added with a 2,4-dinitrophenyl (DNP) group as a tag is also preferable because anti-DNP antibody is commercially available.

Examples of the tagged cholesterol having no linker include fluorescently labeled cholesterol (23-(dipyrrometheneboron difluoride)-24-norcholesterol, CAS No: 878557-19-8) represented by formula (VI) below.

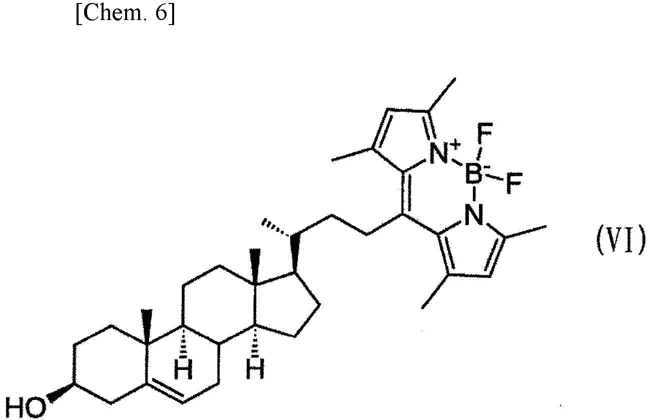

This fluorescently labeled cholesterol is sold by Avanti Polar Lipids, Inc. under the trade name TopFluor Cholesterol. In the fluorescently labeled cholesterol represented by formula (VI), the tag (the fluorescent moiety having a BODIPY backbone structure) is directly bound at the C23 position of cholesterol. As a capture body that specifically binds to the fluorescent moiety having the BODIPY backbone structure, an anti-BODIPY antibody (BODIPY FL Rabbit IgG Fraction, A-5770, Life Technologies Corporation) is commercially available.

The tagged cholesterol in which the tag is bound via a linker includes a biotin-added cholesterol represented by formula (VII) below. 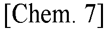 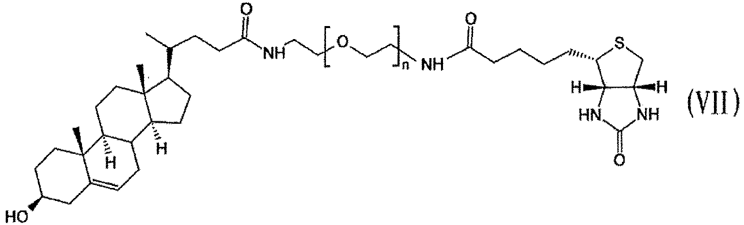 (where n is an integer of 0 to 24, preferably an integer of 2 to 11, and more preferably an integer of 4 to 11.)

In the tagged cholesterol, the tag (the biotin moiety represented by formula (IV)) is bound to the cholesterol moiety via the linker (polyethylene glycol). As a capture body that specifically binds to the biotin moiety, avidin or streptavidin is suitable. Avidin or streptavidin to which a labeling substance such as horseradish peroxidase (HRP) or alkaline phosphatase (ALP) is bound is also commercially available.

The tagged cholesterol in which the tag is bound via a linker includes a DNP-added cholesterol represented by formula (VIII) below.

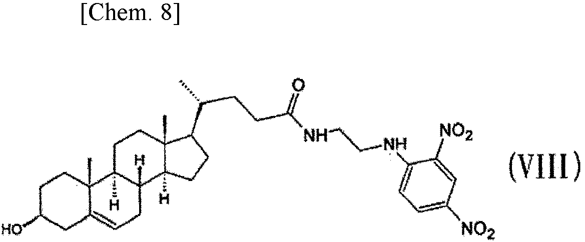

In the tagged cholesterol, DNP is bound to the cholesterol moiety via a linker (-(C=O)-NH-CH₂-CH₂-NH-). As a capture body that specifically binds to DNP, an anti-DNP antibody is suitable. Anti-DNP antibodies to which a labeling substance such as HRP or ALP is bound are also commercially available.

Although the bonding form between the sterol moiety and the tag is not particularly limited, the sterol moiety and the tag may be bound, or the sterol moiety and the tag may be bound via a linker. Although the bonding means is not particularly limited, for example, a crosslink utilizing a functional group is preferable because of convenience. Although the functional group is not particularly limited, an amino group, a carboxyl group, and a sulfhydryl group are preferable because commercially available cross-linkers can be used.

Since cholesterol has no functional group in the alkyl chain bound to the C17 position, a cholesterol derivative having a functional group in the alkyl chain is preferably used in the addition of the tag. Examples of the cholesterol derivative include precursors of bile acids, precursors of steroids, and the like. Specifically, 3β-hydroxy-Δ5-cholenoic acid, 24-amino-5-colen-3β-ol, and the like are preferable. The functional group of the tag varies depending on the type of the tag. For example, when a peptide or a protein is used as the tag, an amino group, a carboxyl group, and a sulfhydryl group (SH group) can be used. When biotin is used as the tag, a carboxyl group in the side chain can be used. The linker is preferably a polymer compound having functional groups at both terminals. When biotin is added as the tag, a commercially available biotin labeling reagent may be used. The reagent contains biotin to which spacer arms (for example, PEG) having various lengths and having a functional group such as an amino group are bound at the terminal.

Examples of the signal generating substance as the first label include fluorescent substances, chromogenic substances, luminescent substances, radioactive isotopes, and the like. Among these, fluorescent substances are particularly preferable. Preferably, the fluorescent substance contains a fluorophore having a polar structure. In this technical field, the fluorescent substance containing a fluorophore having a polar structure is known.

A sterol labeled with a signal generating substance can be produced by adding the substance to a sterol by a known method. In the sterol, the position to which the signal generating substance is added is not particularly limited, and can be determined as appropriate in accordance with the type of the signal generating substance to be used. Although the bonding form of the sterol and the signal generating substance is not particularly limited, it is preferable that both are directly bound via a covalent bond.

Examples of the sterol containing a fluorescent substance include a fluorescently labeled sterol containing a fluorophore having the BODIPY backbone structure represented by formula (IV). In the present embodiment, a commercially available fluorescently labeled sterol may be used. Examples of the sterol containing a fluorophore having the BODIPY backbone structure include the fluorescently labeled cholesterol represented by formula (VI) above. The fluorescently labeled cholesterol represented by formula (VI) generates a fluorescence signal having a wavelength of 525 to 550 nm due to an excitation wavelength of 470 to 490 nm.

### (Preparation of lipoprotein incorporating labeled sterol)

In the present embodiment, mixing of a lipoprotein in a sample and a labeled sterol can be performed, for example, by mixing a sample containing a lipoprotein, a solution containing a labeled sterol, and a solution containing a surfactant having no cyclic structure. The order of the mixing is not particularly limited. After mixing these, the lipoprotein starts to incorporate the labeled sterol. Accordingly, a lipoprotein incorporating the labeled sterol can be prepared. Alternatively, a solution (hereinafter, also referred to as a "reaction buffer") containing a surfactant having no cyclic structure and a labeled sterol may be prepared in advance, and the reaction buffer may be mixed with a sample containing a lipoprotein. When the sample has been diluted with an aqueous medium containing a surfactant having no cyclic structure, the diluted sample and a solution containing a labeled sterol or the reaction buffer may be mixed together. Even when the sample has been diluted by an aqueous medium containing a surfactant having no cyclic structure, a solution containing a surfactant having no cyclic structure or the above-described reaction buffer may be used when bringing the lipoprotein and the labeled sterol into contact with each other.

In the present embodiment, mixing of a lipoprotein in a sample and a labeled sterol may be performed in the presence of the above-described cyclic oligosaccharide and a surfactant having no cyclic structure. The contact between the lipoprotein in the sample and the labeled sterol may be performed in the presence of a surfactant having no cyclic structure and a component that binds to a lipoprotein different from the lipoprotein to be measured. For example, a cyclic oligosaccharide and/or a component that binds to a lipoprotein different from the lipoprotein to be measured may be added to a solution containing a labeled sterol or the reaction buffer.

In the present embodiment, mixing of a lipoprotein in a sample and a labeled sterol may be performed in the presence of a blocking agent for preventing nonspecific adsorption of the labeled sterol. For example, the blocking agent may be added to a solution containing a labeled sterol or the reaction buffer. As the blocking agent, the 2-methacryloyloxyethyl phosphoryl choline-based polymer described above is preferable.

The addition amount of the labeled sterol is not particularly limited, and the labeled sterol may be added in a slightly excessive amount so that the labeled sterol is not depleted. For example, the labeled sterol can be added to a final concentration of not less than 0.1 µM and not higher than 30 µM, and preferably not less than 1 µM and not higher than 10 µM.

The addition amount of the surfactant having no cyclic structure can be set as appropriate in accordance with the type of the surfactant. When the compound represented by formula (I) is used, for example, the addition amount can be set such that the final concentration is not less than 0.001% (v/v) and not higher than 0.5% (v/v), and preferably not less than 0.01% (v/v) and not higher than 0.1% (v/v). When polyoxyethylene lauryl ether is used, for example, the addition amount can be set such that the final concentration is not less than 0.001% (v/v) and not higher than 0.5% (v/v), and preferably not less than 0.01% (v/v) and not higher than 0.1% (v/v).

The conditions of temperature and time in the mixing are not particularly limited. For example, the mixed liquid of the sample and the labeled sterol may be incubated at 20 to 48°C, preferably 25 to 42°C, for 1 minute to 24 hours, preferably 10 minutes to 2 hours. During the incubation, the mixed liquid may be left to stand, or may be stirred or shaken.

### (Formation of complex of lipoprotein and first capture body)

In the measurement method of the present embodiment, it is preferable to further include a step of mixing a lipoprotein and a first capture body that binds to the lipoprotein. Through this mixing, the lipoprotein and the first capture body come into contact with each other, and a complex of the lipoprotein and the first capture body is formed. The first capture body is not particularly limited as long as the first capture body is a substance capable of specifically binding to a part of the surface of the lipoprotein. As the first capture body, an antibody that specifically binds to the lipoprotein is preferable, and an antibody that can specifically bind to apolipoprotein that is a component of lipoprotein is more preferable. Examples of the antibody include anti-ApoAI antibodies, anti-ApoAII antibodies, and the like. Among these, anti-ApoAI antibodies are particularly preferable. Commercially available anti-lipoprotein antibodies and anti-ApoAI antibodies may be used.

The anti-lipoprotein antibodies and the anti-ApoAI antibodies may be monoclonal antibodies or polyclonal antibodies. The origin of the antibody is not particularly limited, and the antibody may be derived from any mammal such as a mouse, a rat, a hamster, a rabbit, a goat, a horse, or a camel. The isotype of the antibody may be any of IgG, IgM, IgE, IgA, and the like and is preferably IgG. In the present embodiment, a fragment of an antibody and a derivative thereof may be used as the first capture body, and examples thereof include a Fab fragment, a F(ab')2 fragment, and the like.

The contact between the lipoprotein and the first capture body can be performed before the contact between the lipoprotein and the labeled sterol, simultaneously with the contact between the lipoprotein and the labeled sterol, or after the contact between the lipoprotein and the labeled sterol. The contact between the lipoprotein and the first capture body can be performed, for example, by mixing the sample containing the lipoprotein, a solution containing the labeled sterol, and a solution containing the first capture body, in the presence of a surfactant having no cyclic structure. The order of mixing the sample containing the lipoprotein, the labeled sterol, and the first capture body is not particularly limited. These may be simultaneously mixed or sequentially mixed in the presence of a surfactant having no cyclic structure. The addition amount of the first capture body is not particularly limited, and can be set as appropriate by a person skilled in the art in in accordance with the type of the first capture body and the like.

In a preferred embodiment, the contact between the lipoprotein and the first capture body is performed after the contact between the lipoprotein and the labeled sterol. As a result, a complex of the lipoprotein incorporating the labeled sterol and the first capture body is formed. For example, first, a sample containing a lipoprotein, and a solution containing a labeled sterol are mixed in the presence of a surfactant having no cyclic structure to prepare a lipoprotein incorporating the labeled sterol. Then, a solution containing the first capture body is mixed thereto. In this case, after the lipoprotein has incorporated the labeled sterol, a complex of the lipoprotein and the first capture body is formed.

When an anti-ApoAI antibody is used as the first capture body, the lipoprotein may be treated with an oxidizing agent before the anti-ApoAI antibody and the lipoprotein incorporating the labeled sterol come into contact with each other. By the action of the oxidizing agent, reactivity between the anti-ApoAI antibody and the lipoprotein can be improved. Examples of the oxidizing agent include hydrogen peroxide, peroxynitrite, chlorine dioxide, hypochlorous acid, and the like.

The conditions of temperature and time in the contact between the lipoprotein and the first capture body are not particularly limited. For example, the mixed liquid described above may be incubated at 20 to 48°C, preferably 25 to 42°C, for 1 minute to 24 hours, preferably 10 minutes to 2 hours. During the incubation, the mixed liquid may be left to stand, or may be stirred or shaken.

In the present embodiment, the complex of the lipoprotein and the first capture body may be formed on a solid phase. For example, the sample containing the lipoprotein, the labeled sterol solution, the solution containing the first capture body, and a solid phase may be mixed in the presence of a surfactant having no cyclic structure. Alternatively, after the sample containing the lipoprotein, the labeled sterol solution, and the solution containing the first capture body are mixed in the presence of the surfactant having no cyclic structure, the obtained mixed liquid may be mixed with a solid phase. Alternatively, the first capture body may be immobilized on a solid phase in advance and used. For example, a solid phase on which an anti-lipoprotein antibody has been immobilized is added to a mixed liquid of the sample containing the lipoprotein and the labeled sterol solution, whereby a complex is formed on the solid phase. The conditions of temperature and time when a solid phase is used are not particularly limited. For example, the conditions may be similar to those for the contact between the lipoprotein and the first capture body.

The solid phase is preferably a solid phase capable of capturing the first capture body in the complex. The type of the solid phase is not particularly limited, and examples thereof include a solid phase of a material that physically adsorbs the antibody, a solid phase on which a molecule that specifically binds to the antibody is immobilized, and the like. Examples of the molecule that specifically binds to the antibody include protein A or G, an antibody (i.e., a secondary antibody) that specifically recognizes the antibody, and the like. A combination of substances interposed between the antibody and the solid phase can be used to bind them. Examples of the combination of substances include combinations of biotin and avidin (or streptavidin), a hapten and an anti-hapten antibody, and the like. For example, when the first capture body is modified with biotin in advance, the first capture body can be captured by a solid phase on which avidin or streptavidin is immobilized.

The solid phase material can be selected from organic polymers, inorganic compounds, biopolymers, and the like. Examples of the organic polymers include latex, polystyrene, polypropylene, styrene-methacrylic acid copolymer, styrene-glycidyl (meth)acrylate copolymer, styrene-styrene sulfonate copolymer, methacrylic acid polymer, acrylic acid polymer, acrylonitrile butadiene styrene copolymer, vinyl chloride-acrylate copolymer, polyvinyl acetate acrylate, and the like. Examples of the inorganic compounds include magnetic bodies (iron oxide, chromium oxide, cobalt, ferrite, etc.), silica, alumina, glass, and the like. Examples of the biopolymers include insoluble agarose, insoluble dextran, gelatin, cellulose, and the like. Two or more types of these may be used in combination. The shape of the solid phase is not particularly limited, and examples thereof include particles, microplates, microtubes, test tubes, and the like. Among these, microplates and particles are preferable, and 96-well microplates and magnetic particles are particularly preferable.

### (Washing step)

In the present embodiment, a washing step of removing unreacted free components may be performed between the contacting step described above and a measurement step described later. This washing step includes B/F separation and washing of the complex. The unreacted free components are components that do not constitute the complex containing the lipoprotein incorporating the labeled sterol. Examples thereof include free labeled sterols that have not been incorporated into the lipoprotein, free first capture bodies that have not bound to the lipoprotein, and the like. The means for B/F separation is not particularly limited, but the complex and the unreacted free components can be separated by collecting only the complex by, for example, ultracentrifugation. In a case where the complex is formed on a solid phase, when the solid phase is particles, the complex and the unreacted free components can be separated by collecting the particles by centrifugation or magnetic separation, and removing the supernatant. When the solid phase is a container such as a microplate or a microtube, the complex and the unreacted free components can be separated by removing the liquid containing the unreacted free components.

After removing the unreacted free components, the collected complex can be washed with an appropriate aqueous medium. Examples of the aqueous medium include water, saline, buffers such as PBS and Tris-HCl, and the like. In the present embodiment, the washing step described above is preferably performed in the presence of a surfactant having no cyclic structure. For example, a surfactant having no cyclic structure may be dissolved in the above-described aqueous medium to prepare a washing liquid, and the collected complex may be washed with the washing liquid. When a solid phase is used, the solid phase having captured the complex may be washed with the washing liquid. Specifically, the washing liquid is added to the collected complex or the solid phase having captured the complex, and B/F separation is performed again.

### (Measurement of uptake capacity)

In the measurement method of the present embodiment, after the preparing step described above, the uptake capacity of the labeled sterol by the lipoprotein is measured. The measurement of the uptake capacity is performed by detecting a signal derived from the labeled sterol incorporated into the lipoprotein. This signal reflects the amount of labeled sterol incorporated into the lipoprotein. Thus, a detection result of the signal serves as an indicator of the uptake capacity of the lipoprotein. Therefore, the measurement method of the present embodiment can also be considered to be a method for evaluating the uptake capacity of the lipoprotein on the basis of a signal derived from the labeled sterol incorporated into the lipoprotein.

Herein, "detecting a signal" includes qualitatively detecting the presence or absence of a signal, quantifying a signal intensity, and semi-quantitatively detecting the intensity of a signal in a plurality of levels, such as "not generating signal", "weak", and "strong".

The signal derived from the labeled sterol incorporated into the lipoprotein may be a signal directly generated from the labeled sterol. The signal can be detected, for example, when a labeled sterol having a signal generating substance as the first label is used. That is, the uptake capacity can be measured by detecting a signal generated from the first label in the labeled sterol incorporated into the lipoprotein. For example, when a fluorescently labeled sterol is used, a fluorescence intensity may be measured. The method for measuring a fluorescence intensity is known in this technical field. For example, the intensity of fluorescence generated from the complex can be measured by using a known measurement apparatus such as a spectrofluorometer and a fluorescence plate reader. The excitation wavelength and the fluorescence wavelength can be determined as appropriate in accordance with the type of the fluorescently labeled cholesterol used. For example, when the fluorescently labeled cholesterol of formula (VI) above is used, the excitation wavelength may be determined from the range of 470 to 490 nm, and the fluorescence wavelength may be determined from the range of 525 to 550 nm.

### (Formation of complex of labeled sterol and second capture body)

The signal derived from the labeled sterol incorporated into the lipoprotein may be a signal generated when the labeled sterol incorporated into the lipoprotein is detected. In order to detect the labeled sterol incorporated into the lipoprotein, in the present embodiment, the labeled sterol may be brought into contact with a second capture body that binds to the labeled sterol, to form a complex. In this case, the labeled sterol is preferably a tagged sterol, and more preferably a tagged cholesterol. The second capture body is preferably a substance that specifically binds to the tag.

The detection principle of the tagged cholesterol incorporated into the lipoprotein is as follows. Usually, when cholesterol is incorporated into a lipoprotein, the cholesterol migrates from the surface layer of the particle of the lipoprotein to the central part thereof. In the tagged cholesterol, it is the cholesterol moiety that is incorporated into the lipoprotein, and the tag is considered to be exposed on the outer surface of the lipoprotein. Here, the "outer surface of the lipoprotein" means the surface on the outer side of the lipoprotein particle. Being "exposed on the outer surface" means both of being present on the outer surface of the lipoprotein and protruding from the outer surface of the lipoprotein. In the present embodiment, the tag exposed on the outer surface is brought into contact with the second capture body that specifically binds to the tag, whereby a complex is formed. Then, cholesterol incorporated into the lipoprotein is detected by detecting the second capture body in this complex.

The substance that specifically binds to the tag can be determined as appropriate in accordance with the type of the tag. For example, with reference to a combination of the above-described tag and a substance capable of specifically binding to the tag, the substance can be selected from antibodies, ligand receptors, oligonucleotides, biotin, avidin (streptavidin), a histidine tag or nickel, GST or glutathione, and the like. Among these, an antibody that specifically binds to the tag is preferable. The antibody may be a commercially available antibody or may be an antibody produced by a method known in the art. The antibody may be a monoclonal antibody or a polyclonal antibody. The origin and isotype of the antibody are not particularly limited, and are similar to those described for the anti-HDL antibody. A fragment of the antibody and a derivative thereof may be used, and examples thereof include a Fab fragment, a F(ab')2 fragment, and the like.

In the present embodiment, it is preferable to bring the labeled sterol and the second capture body into contact with each other, after forming a complex of the lipoprotein incorporating the labeled sterol and the first capture body. In particular, it is preferable to bring the labeled sterol and the second capture body into contact with each other, after forming a complex of the lipoprotein incorporating the labeled sterol and the first capture body on a solid phase. As a result, a complex of the first capture body, the lipoprotein incorporating the labeled sterol, and the second capture body is formed. In this complex, the lipoprotein incorporating the labeled sterol is in a state of being sandwiched by the first capture body and the second capture body. In the present embodiment, the complex of the first capture body, the lipoprotein incorporating the labeled sterol, and the second capture body is also referred to as a "sandwich complex".

The conditions of temperature and time in the contact between the labeled sterol and the second capture body are not particularly limited. For example, the mixture of a solution containing the complex of the lipoprotein and the first capture body and a solution containing the second capture body may be incubated at 4 to 60°C, preferably 25 to 42°C, for 1 second to 24 hours, preferably 10 minutes to 2 hours. During the incubation, the mixture may be left to stand, or may be stirred or shaken.

### (Second label)

The second capture body is preferably labeled with a second label. When the second capture body has been labeled with the second label, the uptake capacity can be measured by detecting a signal derived from the second label in the complex of the labeled sterol and the second capture body.

The second label may be a signal generating substance, or a substance that catalyzes a reaction of another substance to generate a detectable signal can be used. Examples of the signal generating substance include fluorescent substances, radioactive isotopes, and the like. Examples of the substance that catalyzes a reaction of another substance to generate a detectable signal include enzymes. Examples of the enzyme include alkaline phosphatase, peroxidase, β-galactosidase, glucose oxidase, tyrosinase, acid phosphatase, luciferase, and the like. Examples of the fluorescent substance include fluorescent dyes such as fluorescein isothiocyanate (FITC), rhodamine, Alexa Fluor (registered trademark), and cyanine-based dyes, fluorescent proteins such as GFP, and the like. Examples of the radioactive isotope include ¹²⁵I, ¹⁴C, ³²P, and the like. Among these, an enzyme is preferable, and alkaline phosphatase and peroxidase are particularly preferable.

In the present embodiment, labeling of the second capture body with the second label can be performed by directly or indirectly binding the second label to the second capture body. For example, the second label can be directly bound to the second capture body by using a commercially available labeling kit or the like. The second label may be indirectly bound to the second capture body by using a secondary antibody obtained by labeling an antibody capable of specifically binding to the second capture body, with the second label. In the present embodiment, a second capture body to which the second label is bound may be used, or a second capture body and a secondary antibody having the second label may be used.

In the present embodiment, the washing step of removing unreacted free components may be performed before signal detection is performed. Examples of the unreacted free components include free second capture bodies that have not bound to the tag, free second labels that have not bound to the second capture body, and the like. The specific washing method and washing liquid are similar to those in the above-described washing step.

### (Detection of signal derived from second label)

Methods for detecting a signal derived from the second label are known in this technical field. In the present embodiment, an appropriate measurement method can be selected in accordance with the type of the signal derived from the second label. For example, when the second label is an enzyme, signals such as light and color generated through reaction between the enzyme and a substrate for the enzyme can be measured by using a known apparatus. Examples of the measurement apparatus include a spectrophotometer, a luminometer, and the like.

The substrate for the enzyme can be selected as appropriate from known substrates in accordance with the type of the enzyme. For example, when peroxidase is used as the enzyme, examples of the substrate include chemiluminescent substrates such as luminol and derivatives thereof, and chromogenic substrates such as 2,2'-azinobis(3-ethylbenzothiazoline-6-ammonium sulfonate) (ABTS), 1,2-phenylenediamine (OPD) and 3,3',5,5'-tetramethylbenzidine (TMB). When alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate, and p-nitrophenyl phosphate.

When the second label is a radioactive isotope, radiation as a signal can be measured by using a known apparatus such as a scintillation counter. When the second label is a fluorescent substance, fluorescence as a signal can be measured by using a known apparatus such as a fluorescence microplate reader. The excitation wavelength and the fluorescence wavelength can be determined as appropriate in accordance with the type of the fluorescent substance used.

In a preferred embodiment, a method for measuring the uptake capacity of a lipoprotein is provided. The method includes the steps of:
preparing a lipoprotein incorporating a sterol added with a tag, by mixing the lipoprotein in a sample and the sterol added with the tag, in the presence of a surfactant having no cyclic structure;
forming, on a solid phase, a complex of the lipoprotein incorporating the cholesterol added with the tag, and a first capture body that specifically binds to the lipoprotein, in the presence of the surfactant having no cyclic structure;
washing the solid phase with an aqueous medium containing the surfactant having no cyclic structure, thereby removing an unreacted component;
after the washing, forming, on the solid phase, a sandwich complex that contains the lipoprotein incorporating the cholesterol added with the tag, the first capture body, and a second capture body that specifically binds to the tag, by mixing the complex on the solid phase and the second capture body; and
measuring the sandwich complex to measure an uptake capacity of a labeled sterol.

In a preferred embodiment, a method for measuring the uptake capacity of a lipoprotein is provided. The method includes the steps of:
preparing a lipoprotein incorporating a sterol added with a tag, by mixing a surfactant (first surfactant) having no cyclic structure, the lipoprotein in a sample, and the sterol added with the tag;
forming a complex on a solid phase by mixing the lipoprotein incorporating the cholesterol added with the tag, a surfactant (second surfactant) having no cyclic structure, and a first capture body that specifically binds to the lipoprotein;
washing the solid phase with an aqueous medium containing a surfactant (third surfactant) having no cyclic structure, thereby removing an unreacted component;
after the washing, forming, on the solid phase, a sandwich complex that contains the lipoprotein incorporating the cholesterol added with the tag, the first capture body, and a second capture body that specifically binds to the tag, by mixing the complex on the solid phase and the second capture body; and
measuring the sandwich complex to measure an uptake capacity of a labeled sterol.

In the present embodiment, out of the first to third surfactants, at least two surfactants may be of the same type, or the first to third surfactants may be of different types.

In a preferred embodiment, in the step of preparing the lipoprotein incorporating the labeled sterol, a surfactant having a cyclic structure is not substantially contained. In the step of diluting the sample containing the lipoprotein, a surfactant having a cyclic structure is not substantially contained. In the washing step of removing the unreacted free component, a surfactant having a cyclic structure is not substantially contained. That is, in the measurement method of the present embodiment, in each of the steps, it is preferable not to use a surfactant having a cyclic structure. In the present embodiment, even in a case where a slight amount of a surfactant having a cyclic structure and derived from the sample or reagent used is mixed, if there is no influence on the measurement result of the uptake capacity, it is regarded that a surfactant having a cyclic structure is not substantially contained.

### (Method for determining dyslipidemia)

In another embodiment, the result of the uptake capacity of the lipoprotein obtained by the above-described measurement method may be used in determination of whether or not the subject has dyslipidemia. That is, a method of assisting determination of dyslipidemia is provided. The method includes the steps of:
preparing a lipoprotein incorporating a labeled sterol, by mixing the lipoprotein in a sample obtained from a subject and the labeled sterol, in the presence of a surfactant having no cyclic structure;
measuring, on the basis of the labeled sterol incorporated into the lipoprotein, an uptake capacity of the labeled sterol of the lipoprotein; and
obtaining, on the basis of a result of the measurement, information regarding lipid abnormality of the subject.

By accumulating data of signal measurement values obtained from samples of healthy individuals and dyslipidemia patients by the measurement method of the present embodiment, it is possible to determine a threshold or a reference range for the uptake capacity of the lipoprotein. Then, when the threshold or reference range is compared with a signal measurement value obtained by using a sample of a subject, it is possible to obtain information regarding lipid abnormality of the subject, i.e., information of whether or not the uptake capacity of the lipoprotein of the subject is normal or within the reference range. On the basis of this information, it is possible to assist determination of whether or not the subject has dyslipidemia.

### [2. Method 2 for measuring uptake capacity of lipoprotein]

In a measurement method of the present embodiment, a lipoprotein in a sample, a labeled sterol, and a surfactant having no cyclic structure are mixed. As a result of this mixing, a lipoprotein incorporating the labeled sterol is prepared.

The order of mixing the lipoprotein, the labeled sterol, and the surfactant having no cyclic structure is not particularly limited. In an embodiment, in the mixing step, the lipoprotein and the surfactant having no cyclic structure are mixed first, and then, the labeled sterol is added thereto. When the sample containing the lipoprotein is to be diluted, a sample diluting reagent that contains a surfactant having no cyclic structure may be used, or a surfactant having no cyclic structure may be added to the sample before and/or after the dilution.

In another embodiment, in the mixing step, a lipoprotein, and a reagent that contains a labeled sterol and a surfactant having no cyclic structure are mixed. In another embodiment, in the mixing step, a lipoprotein and a labeled sterol are mixed first, and then, a surfactant is added thereto.

In another embodiment, the mixing step includes: a first step of mixing a lipoprotein and a surfactant having no cyclic structure; and a second step of further mixing, to this mixed liquid, a reagent that contains a labeled sterol and a surfactant having no cyclic structure. The surfactant having no cyclic structure used in the first step and the surfactant having no cyclic structure used in the second step may be of the same type or may be of different types.

The other matters of the measurement method of the present embodiment is the same as those described in [1. Method 1 for measuring uptake capacity of lipoprotein].

### [3. Reagent for measuring uptake capacity of lipoprotein]

The scope of the present invention also encompasses a use of a surfactant having no cyclic structure in the above-described measurement method. That is, a reagent for measuring the uptake capacity of a lipoprotein (hereinafter also referred to as "measurement reagent") that contains a labeled sterol and a surfactant having no cyclic structure, is provided. Details of the labeled sterol and the surfactant having no cyclic structure have been described above. The measurement reagent can be used as the reaction buffer described above.

The surfactant having no cyclic structure is preferably selected from nonionic surfactants. As the nonionic surfactant, polyoxyethylene lauryl ether and a polyalkylene glycol ethylene oxide adduct are preferable. As the polyalkylene glycol ethylene oxide adduct, a block copolymer is preferable, and a triblock copolymer of ethylene oxide and propylene oxide is more preferable. As the nonionic surfactant comprising the block copolymer, the compound represented by formula (I) described above is particularly preferable.

As the labeled sterol, the tagged sterol described above, or the sterol having a signal generating substance described above is preferable. Examples of the tagged sterol include the tagged cholesterols represented by formula (III) described above. Among them, the tagged cholesterols represented by formulae (VI), (VII), and (VIII) described above are preferable. Examples of the sterol having a signal generating substance include fluorescently labeled sterols. Examples of the fluorescently labeled sterols include the fluorescently labeled cholesterol represented by formula (VI) described above.

It is preferable that the measurement reagent is in the form of a solution in which the labeled sterol and the surfactant having no cyclic structure are dissolved in an appropriate aqueous medium. The aqueous medium is not particularly limited as long as the aqueous medium does not inhibit the uptake reaction by the lipoprotein. Examples of the aqueous medium include water, saline, buffers such as PBS and Tris-HCl, and the like.

The concentration of the surfactant having no cyclic structure in the measurement reagent may be any concentration that enables, when mixing the measurement reagent and the sample containing the lipoprotein, the final concentration of the surfactant to be not less than 0.001% (v/v) and not higher than 0.5% (v/v), and preferably not less than 0.01% (v/v) and not higher than 0.1% (v/v). Therefore, the concentration of the surfactant having no cyclic structure in the measurement reagent can be determined on the basis of the mixing ratio between the measurement reagent and the sample. In the present embodiment, the concentration of the surfactant having no cyclic structure in the measurement reagent is, for example, not less than 0.002% (v/v) and not higher than 10% (v/v), and preferably not less than 0.005% (v/v) and not higher than 5% (v/v).

The concentration of the labeled sterol in the measurement reagent may be any concentration that enables, when mixing the measurement reagent and the sample containing the lipoprotein, the final concentration of the labeled sterol to be not less than 0.1 µM and not higher than 30 µM, and preferably not less than 1 µM and not higher than 10 µM. Therefore, the concentration of the labeled sterol in the measurement reagent can be determined on the basis of the mixing ratio between the measurement reagent and the sample. In the present embodiment, the concentration of the labeled sterol in the measurement reagent is, for example, not less than 0.2 µM and not higher than 600 µM, and preferably not less than 2 µM and not higher than 200 µM.

The measurement reagent of the present embodiment may contain a cyclic oligosaccharide. Examples of the cyclic oligosaccharide include cyclodextrin, methyl-β-cyclodextrin, hydroxypropyl cyclodextrin, and the like. The concentration of the cyclic oligosaccharide in the measurement reagent is not particularly limited, and is, for example, not less than 0.2 mM and not higher than 20 mM, and preferably not less than 1 mM and not higher than 10 mM.

The measurement reagent of the present embodiment may contain a component that binds to a lipoprotein different from the lipoprotein to be measured. Examples of the component include calixarene and the like. The concentration of the component in the measurement reagent is not particularly limited. When calixarene is used, the concentration is, for example, not less than 0.1 µM and not higher than 0.1 mM, and preferably not less than 1 µM and not higher than 5 µM.

The measurement reagent of the present embodiment may contain a blocking agent for preventing nonspecific adsorption of the labeled sterol. Examples of the blocking agent include a 2-methacryloyloxyethyl phosphoryl choline-based polymer. Details of this polymer have been described above. As the 2-methacryloyloxyethyl phosphoryl choline-based polymer, the LIPIDURE (trademark) series of NOF Corporation is preferable, and LIPIDURE BL-203 is particularly preferable. The concentration of the blocking agent in the measurement reagent is not particularly limited. When the 2-methacryloyloxyethyl phosphoryl choline-based polymer is used, the concentration is, for example, not less than 0.01% (w/w) and not higher than 5% (w/w), and preferably not less than 0.05% (w/w) and not higher than 2% (w/w).

In a preferred embodiment, the measurement reagent does not substantially contain a surfactant having a cyclic structure. In the present embodiment, even in a case where a slight amount of a surfactant having a cyclic structure and derived from a raw material or the like used in preparation of the measurement reagent is mixed, if there is no influence on the measurement result of the uptake capacity, it is regarded that a surfactant having a cyclic structure is not substantially contained.

Also disclosed is the use of the labeled sterol and the surfactant having no cyclic structure for production of the reagent for measuring the uptake capacity of a lipoprotein. That is, the present disclosure also relates to use of the labeled sterol, and the surfactant having no cyclic structure, for production of the reagent for measuring the uptake capacity of a lipoprotein.

### [4. Sample diluting reagent and washing reagent]

Also disclosed is a sample diluting reagent and a washing reagent for use in measurement of the uptake capacity of a lipoprotein. That is, a sample diluting reagent and a washing reagent that contain a surfactant having no cyclic structure are provided. The details of the surfactant having no cyclic structure have been described above.

The sample diluting reagent can be used when diluting a sample containing a lipoprotein, in the measurement method of the present embodiment. Since the sample diluting reagent contains a surfactant having no cyclic structure, when the sample diluted with this reagent and the labeled sterol are mixed, the lipoprotein and the labeled sterol can come into contact with each other in the presence of the surfactant having no cyclic structure.

The washing reagent can be used in the washing step of removing an unreacted free component, in the measurement method of the present embodiment. When the washing reagent containing a surfactant having no cyclic structure is used, collection efficiency of magnetic particles as a solid phase can be improved, as shown in Examples described later.

Preferably, the sample diluting reagent and the washing reagent are each in the form of a solution in which a surfactant having no cyclic structure is dissolved in an appropriate aqueous medium. The aqueous medium is not particularly limited as long as the aqueous medium does not inhibit the uptake reaction of the lipoprotein. Examples of the aqueous medium include water, saline, and buffers such as PBS and Tris-HCl.

The surfactant having no cyclic structure is preferably selected from nonionic surfactants. As the nonionic surfactant, polyoxyethylene lauryl ether and a polyalkylene glycol ethylene oxide adduct are preferable. As the polyalkylene glycol ethylene oxide adduct, a block copolymer is preferable, and a triblock copolymer of ethylene oxide and propylene oxide is more preferable. As the nonionic surfactant comprising the block copolymer, the compound represented by formula (I) described above is particularly preferable. The concentration of the surfactant having no cyclic structure in the sample diluting reagent and the washing reagent is not particularly limited, and is, for example, not less than 0.002% (v/v) and not higher than 10% (v/v), and preferably not less than 0.005% (v/v) and not higher than 5% (v/v).

The sample diluting reagent may contain a cyclic oligosaccharide. Examples of the cyclic oligosaccharide include cyclodextrin, methyl-β-cyclodextrin, hydroxypropyl cyclodextrin, and the like. The concentration of the cyclic oligosaccharide in each reagent is not particularly limited, and is, for example, not less than 0.2 mM and not higher than 20 mM, and preferably not less than 1 mM and not higher than 10 mM.

The sample diluting reagent may contain a component that binds to a lipoprotein different from the lipoprotein to be measured. Examples of the component include calixarene. The concentration of the component in each reagent is not particularly limited. When calixarene is used, the concentration is, for example, not less than 1 µM and not higher than 1 mM, and preferably not less than 10 µM and not higher than 50 µM.

The sample diluting reagent and the washing reagent may each contain a blocking agent for preventing nonspecific adsorption of the labeled sterol. Examples of the blocking agent include a 2-methacryloyloxyethyl phosphoryl choline-based polymer. Details of this polymer have been described above. As the 2-methacryloyloxyethyl phosphoryl choline-based polymer, the LIPIDURE (trademark) series of NOF Corporation is preferable, and LIPIDURE BL-203 is particularly preferable. The concentration of the blocking agent in each reagent is not particularly limited. When the 2-methacryloyloxyethyl phosphoryl choline-based polymer is used, the concentration is, for example, not less than 0.01% (w/w) and not higher than 5% (w/w), and preferably not less than 0.05% (w/w) and not higher than 2% (w/w).

In a preferred embodiment, the sample diluting reagent and the washing reagent do not substantially contain a surfactant having a cyclic structure. In the present embodiment, even in a case where a slight amount of a surfactant having a cyclic structure and derived from a raw material or the like used in preparation of the sample diluting reagent and the washing reagent is mixed, if there is no influence on the measurement result of the uptake capacity, it is regarded that a surfactant having a cyclic structure is not substantially contained.

Also disclosed is a use of the surfactant having no cyclic structure for production of the sample diluting reagent or the washing reagent. That is, the present disclosure also relates to use of the surfactant having no cyclic structure for production of the sample diluting reagent or the washing reagent for use in measurement of the uptake capacity of a lipoprotein.

### [5. Reagent kit for measuring uptake capacity of lipoprotein]

Also disclosed is a reagent kit for use in the measurement method described above. This reagent kit includes the measurement reagent, the sample diluting reagent, and the washing reagent described above. That is, provided is a reagent kit for measuring the uptake capacity of a lipoprotein (hereinafter, also referred to as a reagent kit") that includes: the reagent for measuring the uptake capacity of a lipoprotein containing a labeled sterol and a surfactant having no cyclic structure; the sample diluting reagent containing a surfactant having no cyclic structure; and a washing reagent containing a surfactant having no cyclic structure. Details of the components contained in each reagent have been described above. Among the surfactant having no cyclic structure contained in the reagent for measuring the uptake capacity of a lipoprotein, the surfactant having no cyclic structure contained in the sample diluting reagent, and the surfactant having no cyclic structure contained in the washing reagent, at least two surfactants may be of the same type, or the three surfactants above may be of different types, respectively.

Containers that store the reagents described above may be packaged in a box and provided to a user. This box may include all of the reagents described above, or may include a part of the reagents. The box may include a package insert describing how to use the reagents.

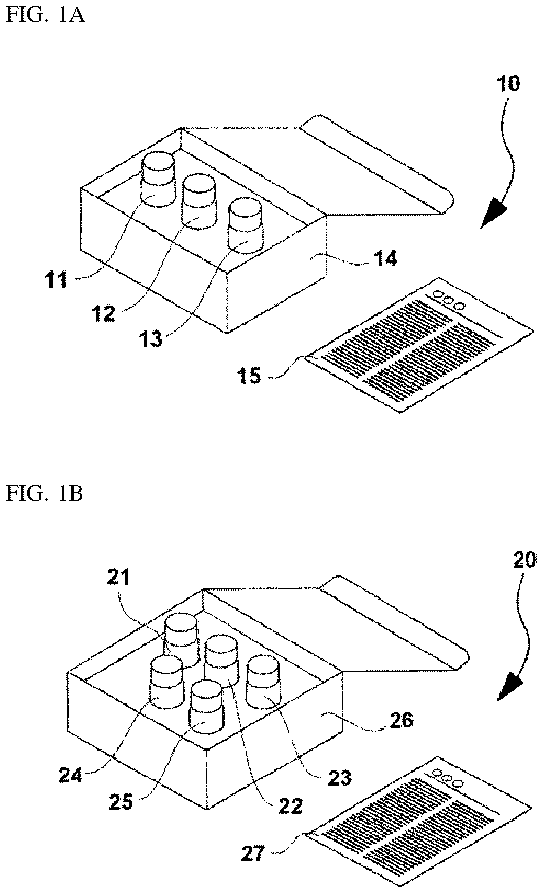

FIG. 1A shows an example of the reagent kit. In FIG. 1A, the reference character 10 represents a reagent kit, the reference character 11 represents a first container storing the measurement reagent, the reference character 12 represents a second container storing the sample diluting reagent, the reference character 13 represents a third container storing the washing reagent, the reference character 14 represents a package box, and the reference character 15 represents a package insert.

The reagent kit may further include a reagent containing the first capture body and a reagent containing the second capture body. The second capture body may be labeled with the second label. FIG. 1B shows an example of a reagent kit. In FIG. 1B, the reference character 20 represents the reagent kit, the reference character 21 represents a first container storing the measurement reagent, the reference character 22 represents a second container storing the sample diluting reagent, the reference character 23 represents a third container storing the washing reagent, the reference character 24 represents a fourth container storing a reagent containing the first capture body, the reference character 25 represents a fifth container storing a reagent containing the second capture body, the reference character 26 represents a package box, and the reference character 27 represents a package insert.

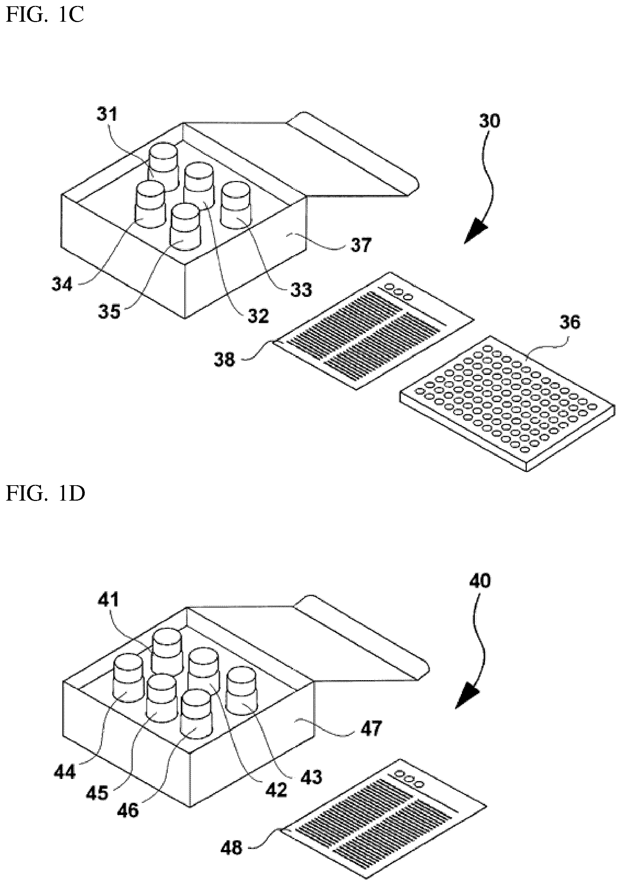

The reagent kit may further include a solid phase for immobilizing the first capture body. The solid phase may be packaged in the box, or may be packaged separately from the box packaging the reagents. FIG. 1C shows an example of a reagent kit further including a solid phase. Details of the solid phase have been described above. In FIG. 1C, the reference character 30 represents the reagent kit, the reference character 31 represents a first container storing the measurement reagent, the reference character 32 represents a second container storing the sample diluting reagent, the reference character 33 represents a third container storing the washing reagent, the reference character 34 represents a fourth container storing a reagent containing the first capture body, the reference character 35 represents a fifth container storing a reagent containing the second capture body, the reference character 36 represents a 96-well microplate as the solid phase, the reference character 37 represents a package box, and the reference character 38 represents a package insert. The solid phase may be magnetic particles. In this case, the reference character 36 in FIG. 1C may be a sixth container storing the magnetic particles.

When the second capture body has been labeled with an enzyme as the second label, the reagent kit may further include a substrate for the enzyme. FIG. 1D shows an example of the reagent kit further including a substrate for the enzyme. Details of the substrate have been described above. In FIG. 1D, the reference character 40 represents the reagent kit, the reference character 41 represents a first container storing the measurement reagent, the reference character 42 represents a second container storing the sample diluting reagent, the reference character 43 represents a third container storing the washing reagent, the reference character 44 represents a fourth container storing a reagent containing the first capture body, the reference character 45 represents a fifth container storing a reagent containing the second capture body labeled with the enzyme, the reference character 36 represents a sixth container storing the substrate for the enzyme, the reference character 47 represents a package box, and the reference character 48 represents a package insert.

In the following, the present invention is described in further detail using Examples. However, the present invention is not limited to these Examples.

### [Examples]

### Example 1: Examination of collection efficiency of magnetic particles

In general, in the ELISA method in which magnetic particles are used as a solid phase, the magnetic particles are washed by using a washing liquid containing a surfactant, after B/F separation is performed. However, in measurement of the cholesterol uptake capacity of a lipoprotein, the surfactant could act on the lipoprotein and influence the measurement result. Therefore, magnetic particles were washed by using a washing liquid containing no surfactant, or a washing liquid containing a Pluronic-type nonionic surfactant.

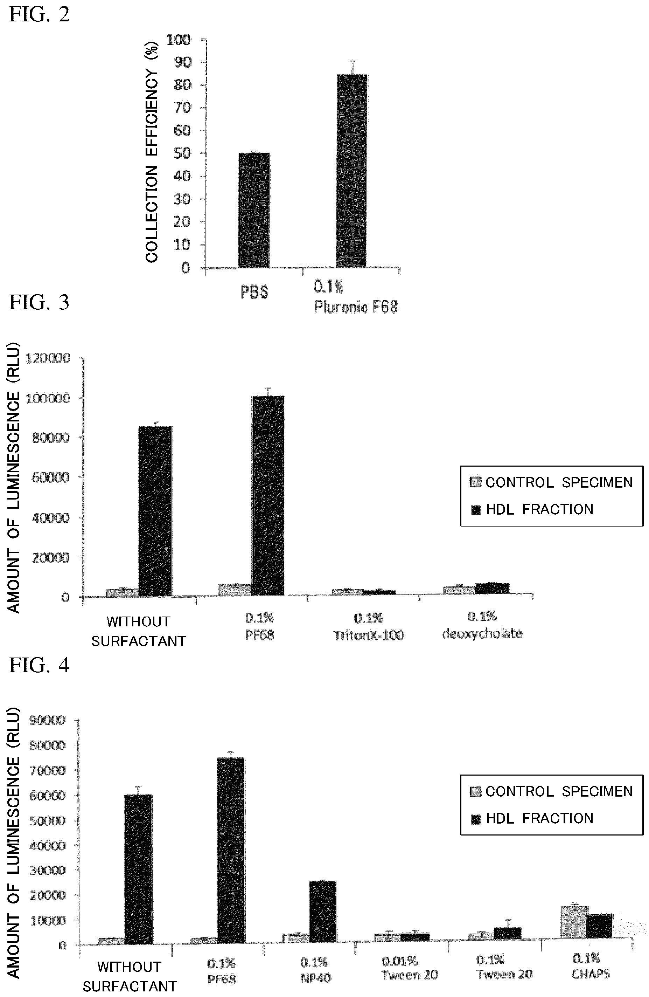

30 µL of 1% HISCL magnetic particles (Sysmex Corporation) was put into a microtube, and washed with PBS or 0.1% (v/v) Pluronic F68/PBS. After the washing, the magnetic particles were collected with a magnet, the supernatant was removed, and the weight of the collected magnetic particles was measured. FIG. 2 shows the collection efficiency of magnetic particles having been washed with each washing liquid.

As shown in FIG. 2, when the magnetic particles were washed with PBS serving as a washing liquid containing no surfactant, the collection efficiency decreased. This revealed that there is another problem in which, when a washing liquid containing no surfactant is used, magnetic particles are attached to the reaction chamber, resulting in decrease of the collection efficiency of the magnetic particles. In contrast to this, when the magnetic particles were washed with a washing liquid containing 0.1% (v/v) Pluronic F68, the collection efficiency was significantly improved.

### Example 2: Examination of influence of surfactant on cholesterol uptake reaction

In order to examine influence of surfactants on the cholesterol uptake capacity of a lipoprotein, the cholesterol uptake capacity was measured by using reaction buffers containing various surfactants. As the surfactant, Pluronic F68 (Thermo), Pluronic L31 (BASF), Pluronic L35 (BASF), Pluronic 10R5 (BASF), Triton X-100 (Sigma-Aldrich), deoxycholic acid (Wako Pure Chemical Industries, Ltd.), NP-40 (Calbiochem), Tween 20 (Sigma-Aldrich), and CHAPS (Dojindo) were used.

### (1) Biological sample

The serum (0.1 mL) of a healthy individual was mixed with an equal amount of 22% polyethylene glycol 4000 (Wako Pure Chemical Industries, Ltd.) to obtain a suspension. The obtained suspension was left to stand at room temperature for 20 minutes, and then centrifuged at 3000 rpm at room temperature for 15 minutes. The obtained supernatant was collected as an HDL fraction.

### (2) Mixing of HDL and tagged cholesterol in the presence of surfactant

### (2.1) Preparation of reaction buffer

Components shown in Table 1 were mixed to prepare a reaction buffer. In Table 1, the units of amount of the respective components are all "µL". In Table 1, the surfactant means any one of Pluronic F68, Pluronic L31, Pluronic L35, Pluronic 10R5, Triton X-100, deoxycholic acid, NP-40, Tween 20, and CHAPS. In Example 2, the final concentration of each component when mixing the reaction buffer and the sample was as follows. Each surfactant: 0.1% (v/v); glycerol: 2% (v/v); LIPIDURE (trademark) BL203: 0.1% (v/v); methyl-β-cyclodextrin: 1 mM; liposome: 0.5% (v/v); and DNP-added cholesterol: 7.5 µM. With respect to some surfactants, the reaction buffer was prepared such that the final concentration was 0.001% (v/v), 0.003% (v/v), 0.01% (v/v), or 0.03% (v/v). The composition of the liposome in Table 1 was 2 mM dimyristoyl phosphatidyl glycerol, 2 mM cholesterol, and 4 mM hydrogenated soybean phosphatidyl choline. PBS was prepared by dissolving Phosphate buffered saline tablet (Sigma-Aldrich) in water. The DNP-added cholesterol was prepared with reference to US 2017/0315112 A1.

**[Table 1]**

| Component | Without surfactant | With surfactant |
|---|---|---|
| PBS | 953 | 941 |
| 50% glycerol | 48 | 48 |
| Surfactant | 0 | 12 |
| LIPIDURE (trademark) -BL203 | 1.2 | 1.2 |
| 20 mM methyl-β-cyclodextrin | 60 | 60 |
| Liposome | 6 | 6 |
| 0.75 mM DNP-added cholesterol | 12 | 12 |
| Total | 1080 | 1080 |

### (2.2) Preparation of measurement sample

### (i) Dilution of biological sample

A fraction liquid included in HDL-C reagent·KL "kokusai" (Sysmex Corporation) was diluted to 1.85% with PBS, to prepare a sample diluting reagent. This sample diluting reagent includes cyclodextrin, calixarene, and NONION K-230 derived from the fraction liquid included in HDL-C reagent·KL "kokusai". A part of the HDL fraction above was taken, and the ApoAI concentration was measured by using an ApoAI measurement kit (N-assay TIA ApoAI-H, NITTOBO MEDICAL Co., LTD.). A specific concentration measurement operation was performed in accordance with the manual attached to the kit. After the measurement, the HDL fraction was diluted with the sample diluting reagent, to prepare a HDL fraction-containing dilution liquid having an ApoAI concentration of 1,000 ng/mL. As a control specimen (ApoAI concentration: 0 ng/mL) containing no HDL fraction, the sample diluting reagent was used.

### (ii) Mixing of HDL and tagged cholesterol

The HDL fraction-containing dilution liquid (120 µL) was added to each of a reaction buffer (1080 µL) containing a surfactant and a reaction buffer (1080 µL) containing no surfactant, and the resultant matter was mixed. Then, the mixture was left to stand on ice for 1 hour. 500 µL of the obtained mixture was taken and transferred into a 1.5 mL low-binding tube, and the tube was shaken at 42°C at 1,000 rpm for 20 minutes. Accordingly, HDL and the DNP-added cholesterol were brought into contact with each other under the surfactant, whereby the uptake reaction of the DNP-added cholesterol by HDL was promoted. Then, the tube was left to stand at room temperature for 5 minutes, to obtain a measurement sample. The control specimen was treated in the same manner.

### (3) Measurement of cholesterol uptake capacity by HDL

### (3.1) Preparation of measurement plate (immobilization of anti-ApoAI antibody to solid phase)

200 µL of 50 mM Tris-HCl (pH 7.5) was added to each well of a 96-well microplate (black plate H for fluorescence measurement, manufactured by Sumitomo Bakelite Co., Ltd.) as a solid phase for washing. This washing operation was performed twice in total. 100 µL of a solution of an anti-ApoAI antibody (clone 8E10) diluted to a concentration of 10 µg/mL with 50 mM Tris-HCl (pH 7.5) was added to each well, and the well was left to stand at 4°C overnight. The antibody solution was removed, and 200 µL of 138 mM NaCl aqueous solution was added to each well for washing. This washing operation was performed three times in total. 200 µL of 2% BSA/PBS was added to each well, and the microplate was shaken at 25°C at 600 rpm for 1 hour.

### (3.2) Formation of complex of HDL and anti-ApoAI antibody on solid phase

The BSA solution was removed from the plate on which the anti-ApoAI antibody was immobilized, and 100 µL of each measurement sample was added to the wells. Then, the plate was shaken at 42°C at 1,000 rpm for 20 minutes, to form a complex of HDL and the anti-ApoAI antibody. The measurement sample was removed from each well, and 200 µL of a line washing liquid containing 138 mM NaCl and 0.1% Pluronic F68 was added to wash the well. This washing operation was performed three times in total. In Example 1, two plates in which the complex was captured in this manner were prepared.

### (3.3) Detection of tagged cholesterol incorporated into HDL

An anti-DNP antibody (produced by KITAYAMA LABES CO., LTD. on commission) was labeled with ALP by using ALP Labeling Kit-SH (Dojindo), to prepare an ALP labeled anti-DNP antibody. The obtained ALP labeled anti-DNP antibody was diluted with HISCL R3 buffer (Sysmex Corporation) to 1:500. In one of the two plates described above, 100 µL of the dilution liquid of the ALP labeled anti-DNP antibody was added to each well. The plate was shaken at 25°C at 600 rpm for 1 hour. The antibody solution was removed from each well, and 200 µL of a line washing liquid containing 138 mM NaCl and 0.1% Pluronic F68 was added to the well for washing. This washing operation was performed three times in total. 100 µL of a substrate solution for ALP was added to each well. The substrate solution was prepared by mixing an R4 reagent and an R5 reagent, which are HISCL luminescent substrates (Sysmex Corporation), at a ratio of 1:2. The plate was shaken at 25°C at 600 rpm for 30 minutes, and then, the amount of luminescence was measured by a microplate reader (Infinite (registered trademark) F200 Pro, manufactured by TECAN). The measurement results are shown in FIGS. 3 to 6. "PF68" in FIG. 3 and FIG. 4 means Pluronic F68.

### (3.4) Measurement of the amount of captured complex

In the other of the prepared two plates, 100 µL of a solution of an ALP labeled anti-ApoAI antibody (clone PIA5) diluted to 1:8000 was added to each well. The plate was shaken at 25°C at 600 rpm for 1 hour. The antibody solution was removed from each well, and 200 µL of a line washing liquid containing 138 mM NaCl and 0.1% Pluronic F68 was added to wash the well. This washing operation was performed three times in total. 100 µL of a substrate solution for ALP was added to each well. The plate was shaken at 25°C at 600 rpm for 30 minutes, and then the amount of luminescence was measured by a microplate reader (Infinite (registered trademark) F200 Pro, manufactured by TECAN). Although the measurement results are not shown, no significant difference was observed in the amount of the captured complex, between the specimens.

### (4) Result

As shown in FIGS. 3 and 4, when a reaction buffer containing Triton X-100, deoxycholic acid, NP-40, Tween 20, or CHAPS was used, the signal (black bar) indicating the cholesterol uptake capacity was significantly reduced. Although these surfactants are surfactants having cyclic structures generally used in immunoassays, the signals decreased to the same levels as those of the background (gray bar) in almost all the surfactants. Conceivable reasons for this are that the surfactant acted on the lipoprotein and the labeled cholesterol to inhibit the uptake reaction, or that the incorporated cholesterol leaked.

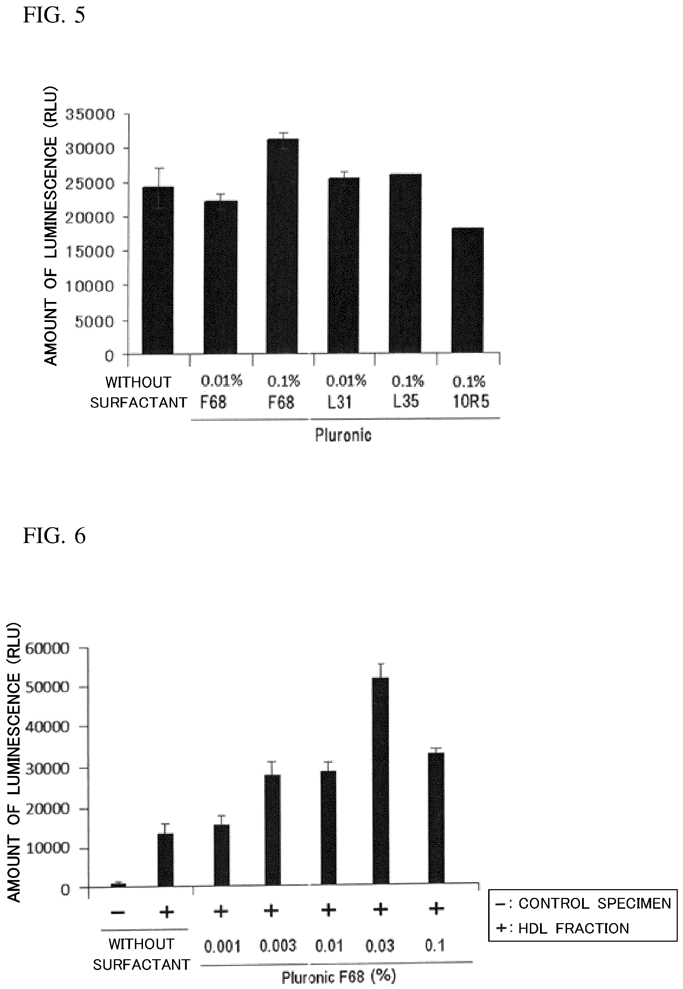

Meanwhile, as shown in FIGS. 3 to 5, when a reaction buffer containing Pluronic F68, Pluronic L31, Pluronic L35, or Pluronic 10R5, which are surfactants having no cyclic structure, was used, a signal that indicates an uptake capacity equivalent to that when a reaction buffer containing no surfactant was used, was obtained. FIG. 6 revealed that, when a reaction buffer containing Pluronic F68 was used, the signal indicating the uptake capacity was enhanced, when compared with cases where a reaction buffer containing no surfactant was used.
"Without surfactant" in FIGS. 3 to 6 indicates that a reaction buffer containing no surfactant was used. Although these reaction buffers contained no surfactant, the diluted sample contained NONION K-230 which is a surfactant having no cyclic structure. This NONION K-230 is derived from the sample diluting reagent. Therefore, with respect to "without surfactant", mixing of HDL and the tagged cholesterol was performed in the presence of a surfactant having no cyclic structure.

Thus, it was shown that, by mixing a lipoprotein and a labeled cholesterol in the presence of a surfactant having no cyclic structure, it is possible to favorably detect the signal indicating the cholesterol uptake capacity by the lipoprotein.

### Example 3: Application of surfactant to sample diluting reagent

In Example 2, even when a reaction buffer containing no surfactant was used, the signal indicating the cholesterol uptake capacity by HDL was favorably detected. Here, in Example 2, NONION K-230, which is a nonionic surfactant having no cyclic structure was contained in the reagent for diluting the sample containing a lipoprotein. Therefore, in order to examine influence of surfactants in dilution of the sample, the cholesterol uptake capacity was measured by using sample diluting reagents containing various surfactants. As the surfactant having no cyclic structure, NONION K-230 (manufactured by NOF Corporation), Pluronic L31, Pluronic L61, and Pluronic P84 (each manufactured by BASF) were used. As a surfactant having a cyclic structure, Triton X-100 (manufactured by Sigma-Aldrich) and Tween 20 (manufactured by Sigma-Aldrich) were used. Measurement of the uptake capacity was performed in accordance with the ELISA method using magnetic particles as a solid phase.

### (1) Biological sample

From a mixture of five clinical specimens (serum) having high HDL-cholesterol values, an HDL fraction was prepared in a manner similar to that in Example 2.

### (2) Mixing of HDL and tagged cholesterol in the presence of surfactant

### (2.1) Preparation of sample diluting reagent

As a sample diluting reagent, PBS that contains 0.01% (v/v) or 0.1% (v/v) NONION K-230, 0.1% (v/v) Pluronic L31, 0.01% (v/v) or 0.1% (v/v) Pluronic L61, 0.01% (v/v) Pluronic P84, 0.1% (v/v) Tween 20, or 0.1% (v/v) Triton X-100, was prepared.

### (2.2) Preparation of measurement sample

### (i) Dilution of biological sample

A part of the HDL fraction above was taken, and the ApoAI concentration was measured in the same manner as in Example 2. After the measurement, the HDL fraction was diluted with each sample diluting reagent, to prepare an HDL fraction-containing dilution liquid having an ApoAI concentration of 450 ng/mL. As a control sample (ApoAI concentration: 0 ng/mL) containing no HDL fraction, the sample diluting reagent was used.

### (ii) Mixing of HDL and tagged cholesterol

In a similar manner to that in Example 2, a reaction buffer containing 0.1% (v/v) Pluronic F68 and 7.5 µM DNP-added cholesterol was prepared. The reaction buffer (60 µL) and the HDL fraction-containing dilution liquid (10 µL) were added to a cuvette and mixed, and the cuvette was left to stand on ice for 30 minutes. Then, the cuvette was left to stand at 42°C for 1 minute, to obtain a measurement sample. The control sample was treated in the same manner.

### (3) Measurement of cholesterol uptake capacity by HDL

### (3.1) Formation of complex of HDL and anti-ApoAI antibody on magnetic particle

An anti-ApoA1 antibody (clone 8E10) was reduced with 2-mercaptoethylamine hydrochloride (NACALAI TESQUE), and labeled with Biotin-PEAC5-maleimide (Dojindo), whereby a biotinylated ApoAI antibody was prepared. 30 µL of 2.5 ng/µL biotinylated ApoAI antibody was added to a cuvette storing the reaction buffer and the HDL fraction-containing dilution liquid, and the resultant matter was reacted at 42°C for 12 minutes. After the reaction, 30 µL of HISCL magnetic particles (Sysmex Corporation) was added to the cuvette, and the resultant matter was reacted at 42°C for 10 minutes. Since avidin is immobilized on the surface of the HISCL magnetic particle, the biotinylated anti-ApoAI antibody in the complex binds to the surface of the magnetic particle. The magnetic particles in the reaction liquid were collected with a magnet, the supernatant was removed, and then 0.1% (v/v) Pluronic F68/PBS was added to wash the magnetic particles. This washing operation was performed three times in total. Then, the magnetic particles were collected with a magnet and the supernatant was removed.

### (3.2) Detection of tagged cholesterol incorporated into HDL

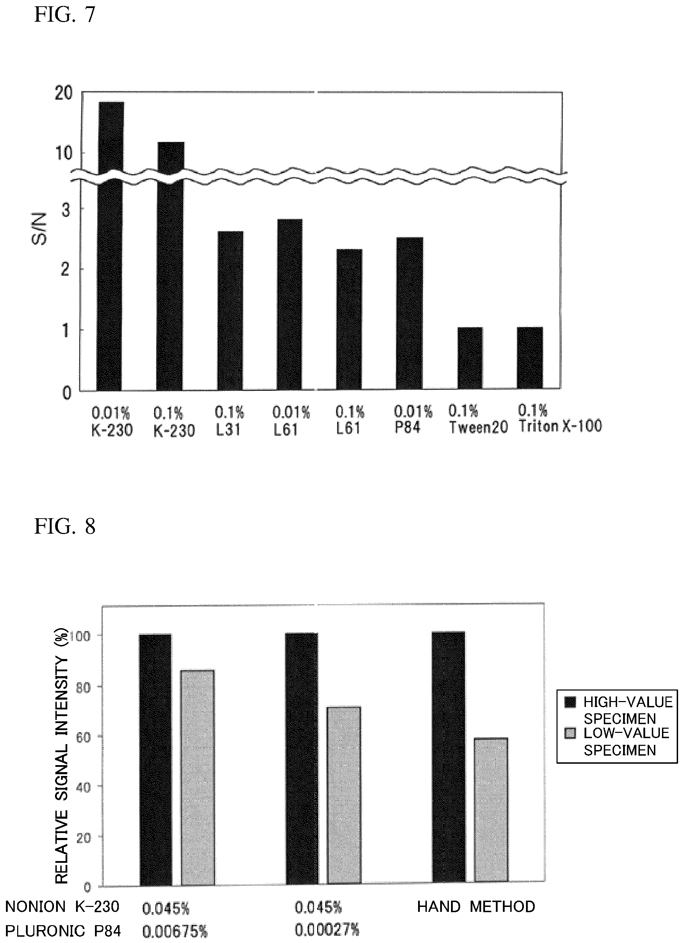

100 µL of the same dilution liquid of the ALP labeled anti-DNP antibody as that in Example 2 was added to the magnetic particles having HDL incorporating the DNP-added cholesterol, and the resultant matter was reacted at 42°C for 10 minutes. The magnetic particles in the reaction liquid were collected with a magnet, the supernatant was removed, and 0.1% (v/v) Pluronic F68/PBS was added to wash the magnetic particles. This washing operation was performed three times in total. The magnetic particles were transferred to another cuvette, the magnetic particles were collected with a magnet, and the supernatant was removed. The HISCL R4 reagent (50 µL) and R5 reagent (100 µL) were added to the cuvette storing the magnetic particles, and the resultant matter was reacted at 42°C for 5 minutes. After the reaction, the amount of luminescence was measured. The ratio (S/N) between the measurement value (signal) obtained from the sample containing the HDL fraction and the measurement value (noise) obtained from the control sample was calculated. The results are shown in FIG. 7.

### (4) Result

As shown in FIG. 7, when a sample diluting reagent containing Tween 20 or Triton X-100 was used, there was substantially no difference between the signal and the noise, and S/N was about 1. Thus, the cholesterol uptake capacity of HDL could not be evaluated. Meanwhile, when a sample diluting reagent containing NONION K-230, Pluronic L31, Pluronic L61, or Pluronic P84 was used, S/N was greater than that obtained when a sample diluting reagent containing Tween 20 or Triton X-100 was used. Therefore, it was shown that by adding a surfactant having no cyclic structure to the sample diluting reagent, S/N of the uptake capacity measurement can be improved.

### Example 4: Comparison with hand method

Correlation between a measurement method (hereinafter, referred to as "hand method") for the uptake capacity based on a microplate using a BSA solution as a sample diluting reagent and a measurement method based on magnetic particles using a sample diluting reagent containing a surfactant was examined.

### (1) Biological sample

As the biological sample, two types of HDL fractions of which the cholesterol uptake capacity was measured in advance by the hand method were used. One of them is an HDL fraction (hereinafter, referred to as "high-value specimen") that indicated a high signal by the hand method, and the other is an HDL fraction (hereinafter, referred to as "low-value specimen") that indicated a low signal by the hand method.

### (2) Measurement of cholesterol uptake capacity by ELISA method using magnetic particle

### (2.1) Preparation of sample diluting reagent

The components shown in Table 2 were dissolved in PBS to prepare sample diluting reagents A to G. In Table 2, "HP-γ-CD" is hydroxypropyl-γ-cyclodextrin (NIHON SHOKUHIN KAKO CO., LTD.).

**[Table 2]**

| Component | Reagent A | Reagent B | Reagent C | Reagent D | Reagent E | Reagent F | Reagent G |
|---|---|---|---|---|---|---|---|
| NONION K-230 (% (v/v) | 0.045 | 0.045 | 0.03 | 0.03 | 0.03 | 0.03 | 0.03 |
| Pluronic P84 (% (v/v)) | 0.00675 | 0.00027 | 0.00675 | 0.00675 | 0.00675 | 0.00675 | 0.00675 |
| HP-γ-CD (% (w/v)) | - | - | - | 0.0015 | 0.075 | 0.0375 | 0.1875 |

### (2.2) Preparation of measurement sample

### (i) Dilution of biological sample

A part of each of the high-value specimen and the low-value specimen described above was taken, and the ApoAI concentration was measured in the same manner as in Example 2. After the measurement, the high-value specimen and the low-value specimen were each diluted by each sample diluting reagent, to prepare an HDL fraction-containing dilution liquid having an ApoAI concentration of 450 ng/mL. As a control sample (ApoAI concentration: 0 ng/mL) containing no HDL fraction, the sample diluting reagent was used.

### (ii) Mixing of HDL and tagged cholesterol

In a similar manner to that in Example 2, a reaction buffer containing 0.1% (v/v) Pluronic F68 and 7.5 µM DNP-added cholesterol was prepared. In a similar manner to that in Example 3, the reaction buffer and the HDL fraction-containing dilution liquid were mixed, to obtain a measurement sample. The control sample was treated in the same manner.

### (2.3) Measurement of cholesterol uptake capacity by HDL

### (i) Formation of complex of HDL and anti-ApoAI antibody on magnetic particle

Similar to Example 3, a biotinylated ApoAI antibody was added to a cuvette storing the reaction buffer and the HDL fraction-containing dilution liquid, and the resultant matter was reacted at 42°C for 12 minutes. After the reaction, 30 µL of the HISCL magnetic particles (Sysmex Corporation) was added to the cuvette, and the resultant matter was reacted at 42°C for 10 minutes. The magnetic particles in the reaction liquid were collected with a magnet, the supernatant was removed, and 0.1% (v/v) Pluronic F68/PBS was added to wash the magnetic particles. This washing operation was performed three times in total. Then, the magnetic particles were collected with a magnet and the supernatant was removed.

### (ii) Detection of tagged cholesterol incorporated into HDL

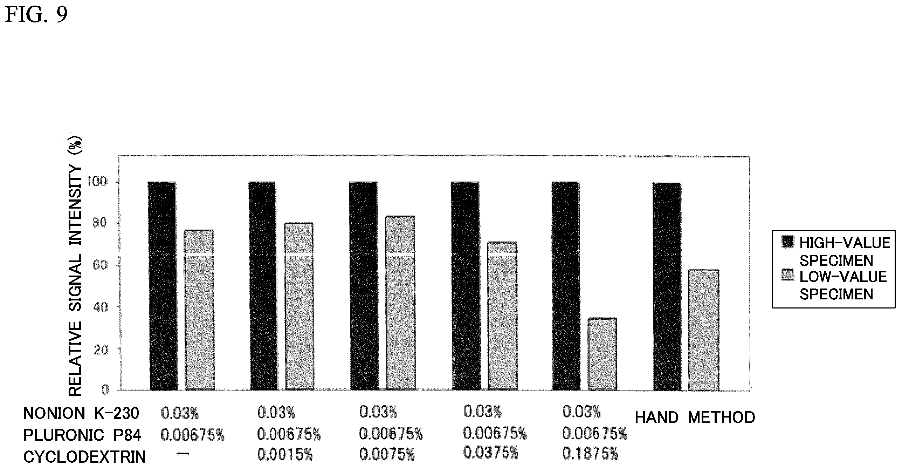

Similar to Example 3, the dilution liquid of the ALP labeled anti-DNP antibody was added to the magnetic particles having HDL incorporating the DNP-added cholesterol, and the resultant matter was reacted at 42°C for 10 minutes. The magnetic particles in the reaction liquid were collected with a magnet, the supernatant was removed, and 0.1% (v/v) Pluronic F68/PBS was added to wash the magnetic particles. This washing operation was performed three times in total. The magnetic particles were transferred to another cuvette, the magnetic particles were collected with a magnet, and the supernatant was removed. Similar to Example 3, the HISCL R4 reagent and R5 reagent were added to the cuvette storing the magnetic particles, and the resultant matter was reacted. After the reaction, the amount of luminescence was measured. The proportion (relative signal intensity) of the measurement value of the low-value specimen when the measurement value of the high-value specimen was assumed to be 100% was calculated. The results are shown in FIGS. 8 and 9.

### (3) Measurement of cholesterol uptake capacity by hand method

### (3.1) Preparation of measurement sample

### (i) Dilution of biological sample

In the hand method, 2% BSA/PBS was used as the sample diluting reagent. Each of the high-value specimen and the low-value specimen described above was diluted with 2% BSA/PBS, to prepare an HDL fraction-containing dilution liquid having an ApoAI concentration of 450 ng/mL. As a control specimen (ApoAI concentration: 0 ng/mL) containing no HDL fraction, 2% BSA/PBS was used.

### (ii) Mixing of HDL and tagged cholesterol

In the same manner as in Example 2 except that a BODIPY-added cholesterol (TopFluor Cholesterol, AvantiPolar Lipids), instead of the DNP-added cholesterol, was used as the reaction buffer, a reaction buffer containing 0.1% (v/v) Pluronic F68 and 7.5 µM BODIPY-added cholesterol was prepared. Similar to Example 2, the reaction buffer and the HDL fraction-containing dilution liquid were mixed, to obtain a measurement sample. The control specimen was treated in the same manner.

### (3.2) Measurement of cholesterol uptake capacity by HDL

### (i) Formation of complex of HDL and anti-ApoAI antibody on plate

In a similar manner to that in Example 2, a 96-well microplate on which the anti-ApoAI antibody was immobilized was prepared. Similar to Example 2, each measurement sample was added to each well, to form a complex of HDL and the anti-ApoAI antibody on the plate. Washing of the well was performed in the same manner as in Example 2, by using a line washing liquid containing 138 mM NaCl and 0.1% Pluronic F68.

### (3.3) Detection of tagged cholesterol incorporated into HDL

100 µL of 10 mM cyclodextrin/PBS was added to each well having been washed, and the plate was shaken at 25°C at 600 rpm for 15 minutes. The plate was set to a fluorescence plate reader (Infinite (registered trademark) 200 Pro, manufactured by TECAN), and fluorescence intensity was measured (excitation light 485 nm/fluorescence 535 nm). Relative signal intensity was calculated from the measurement values of the high-value specimen and the low-value specimen. The results are shown in FIGS. 8 and 9.

### (4) Result

FIG. 8 revealed that, in the case of the hand method, the cholesterol uptake capacity of the low-value specimen was lower than the cholesterol uptake capacity of the high-value specimen. Also in the case of the measurement method based on magnetic particles using a sample diluting reagent containing NONION K-230 and Pluronic P84, results similar to those by the hand method were obtained. As shown in FIG. 9, it was found that the correlation with the hand method was improved by using a sample diluting reagent further containing cyclodextrin.

### DESCRIPTION OF THE REFERENCE CHARACTERS

10, 20, 30, 40: reagent kit
11, 21, 31, 41: first container
12, 22, 32, 42: second container
13, 23, 33, 43: third container
24, 34, 44: fourth container
25, 35, 45: fifth container
36: solid phase
46: sixth container
14, 26, 37, 47: package box
15, 27, 38, 48: package insert

## Claims

1. A method for measuring an uptake capacity of a lipoprotein, the method comprising the steps of:
preparing a lipoprotein incorporating a labeled sterol, by mixing the lipoprotein in a sample and the labeled sterol in the presence of a surfactant having no cyclic structure; and
measuring an uptake capacity of the labeled sterol on the basis of a label of the labeled sterol incorporated into the lipoprotein,
wherein the surfactant having no cyclic structure is a polyalkylene glycol ethylene oxide adduct and/or polyoxyethylene lauryl ether.

2. The method according to claim 1, wherein the surfactant is a nonionic surfactant.

3. The method according to claim 1, wherein the polyalkylene glycol ethylene oxide adduct is of a block copolymerization type.

4. The method according to claim 3, wherein
the surfactant of the block copolymerization type is a compound represented by formula (I) below: 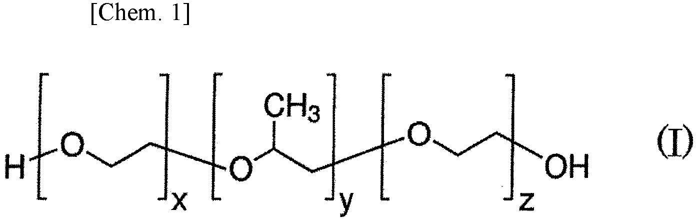 (where x, y, and z are identical or different from each other, and are each an integer not smaller than 1, and
a molecular weight of a polypropylene oxide block is not greater than 2750), or
a compound represented by formula (II) below: 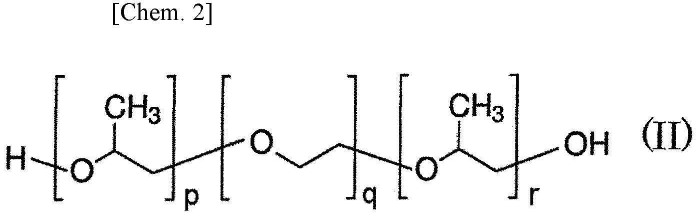 (where p, q, and r are identical or different from each other, and are each an integer not smaller than 1, and
a molecular weight of a polypropylene oxide moiety is not greater than 2750).

5. The method according to any one of claims 1 to 4, wherein
the labeled sterol is a tagged cholesterol represented by formula (III) below: 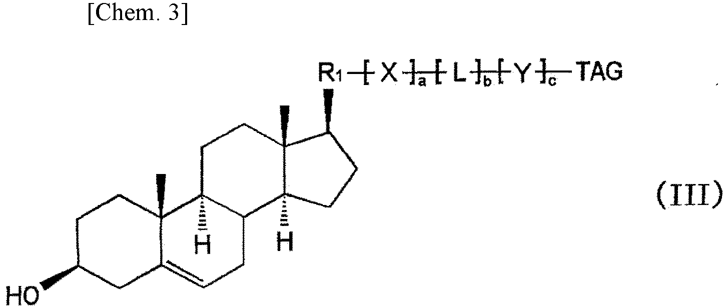 (where R₁ is an alkylene group having 1 to 6 carbon atoms and optionally having a methyl group,
X and Y are identical or different from each other, and are each represented by - R₂-NH-, -NH-R₂-, -R₂-(C=O)-NH-, -(C=O)-NH-R₂-, -R₂-NH-(C=O)-, -NH-(C=O)-R₂-, -R₂-(C=O)-, -(C=O)-R₂-, -R₂-(C=O)-O-, -(C=O)-O-R₂-, -R₂-O-(C=O)-, -O-(C=O)-R₂-, -R₂-(C=S)-NH-, -(C=S)-NH-R₂-, -R₂-NH-(C=S)-, -NH-(C=S)-R₂-, -R₂-O-, -O-R₂-, -R₂-S-, or -S-R₂-, and each R₂ is independently: an atomic bonding; an alkylene group having 1 to 10 carbon atoms and optionally having a substituent; an arylene group or heteroarylene group having 6 to 12 carbon atoms and optionally having a substituent; or a cycloalkylene group or heterocycloalkylene group having 3 to 8 carbon atoms and optionally having a substituent,
L is represented by -(CH₂)_{d}-[R₃-(CH₂)ₑ]_{f}- or -[(CH₂)ₑ-R₃]_{f}-(CH₂)_{d}-, and R₃ is an oxygen atom, a sulfur atom, -NH-, -NH-(C=O)-, or -(C=O)-NH-,
TAG is a tag,
a and c are identical or different from each other, and are each an integer of 0 to 6,
b is 0 or 1,
d and e are identical or different from each other, and are each an integer of 0 to 12, and
f is an integer of 0 to 24.)

6. The method according to claim 5, wherein
the tagged cholesterol comprises, as the tag, a structure added with
- a structure represented by formula (IV) below: 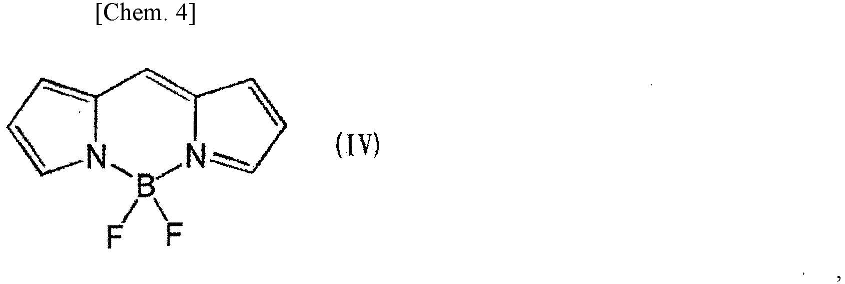
- a structure represented by formula (V) below: 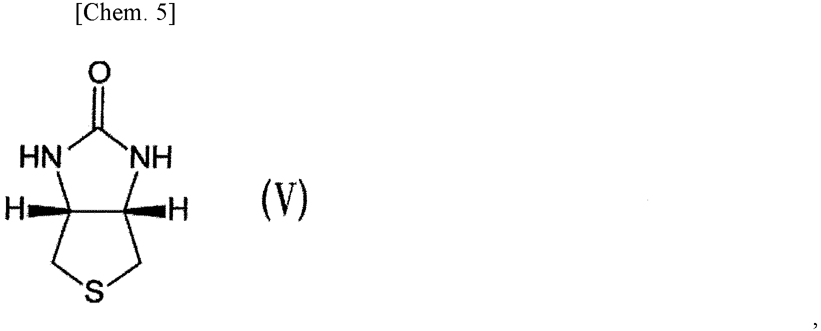 or
- a 2,4-dinitrophenyl group.

7. The method according to claim 6, wherein
the tagged cholesterol is represented by formula (VI) below: 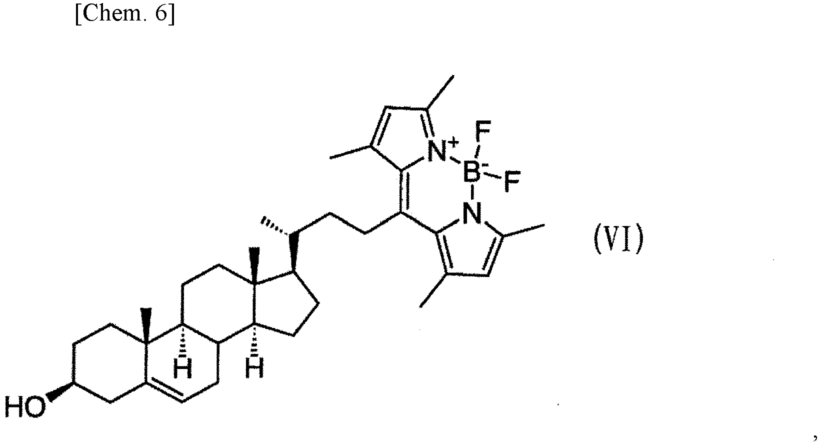
or formula (VII) below: 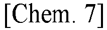 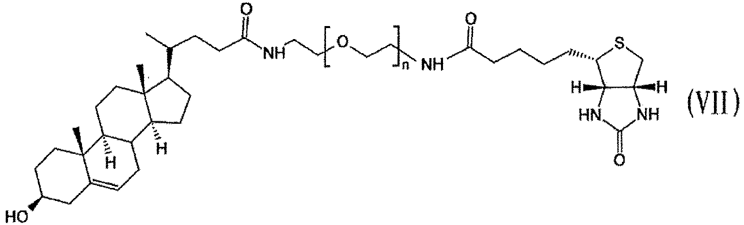 (where n is an integer of 0 to 24.),
or formula (VIII) below:

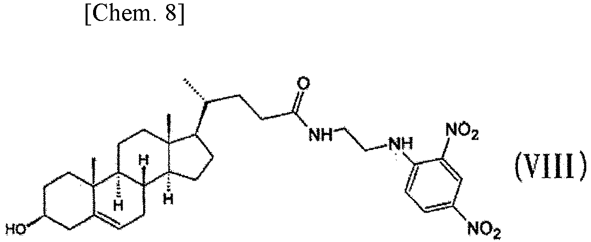

8. The method according to any one of claims 1 to 7, wherein
the measuring comprises mixing the lipoprotein incorporating the labeled sterol and a first capture body that binds to the lipoprotein, to form a complex that contains the lipoprotein incorporating the labeled sterol and the first capture body.

9. The method according to any one of claims 1 to 8, wherein
the preparing is performed in the presence of a 2-methacryloyloxyethyl phosphoryl choline-based polymer.

10. The method according to any one of claims 1 to 9, wherein
the sample is a sample diluted, before the preparing, with an aqueous medium containing a surfactant having no cyclic structure.

11. The method according to claim 10, wherein
the dilution is performed in the presence of a cyclic oligosaccharide and/or a component that binds to a lipoprotein different from a lipoprotein to be measured.

12. The method according to claim 11, wherein
the cyclic oligosaccharide is cyclodextrin, methyl-β-cyclodextrin, or hydroxypropyl cyclodextrin, and the component that binds to the lipoprotein different from the lipoprotein to be measured is calixarene.

13. The method according to any one of claims 1 to 12, further comprising
between the preparing and the measuring, washing in which an unreacted free component is removed in the presence of a surfactant having no cyclic structure.

14. Use of a surfactant having no cyclic structure for the method according to any of claims 1 to 13.

## Patentansprüche

1. Verfahren zum Messen einer Aufnahmekapazität eines Lipoproteins, wobei das Verfahren die Schritte umfasst:
Erzeugen eines Lipoproteins, welches ein markiertes Sterin inkorporiert, durch Mischen des Lipoproteins in einer Probe und des markierten Sterins in der Gegenwart eines Tensids, welches keine zyklische Struktur aufweist; und
Messen einer Aufnahmekapazität des markierten Sterins auf Grundlage einer Markierung des in das Lipoprotein inkorporierten markierten Sterins,
wobei das Tensid, welches keine zyklische Struktur aufweist, ein Polyalkylenglycol-Ethylenoxid-Addukt und/oder Polyoxyethylenlaurylether ist.

2. Verfahren gemäß Anspruch 1, wobei das Tensid ein nichtionisches Tensid ist.

3. Verfahren gemäß Anspruch 1, wobei das Polyalkylenglycol-Ethylenoxid-Addukt vom Blockcopolymerisationstyp ist.

4. Verfahren gemäß Anspruch 3, wobei
das Tensid des Blockcopolymerisationstyps eine Verbindung ist, dargestellt durch Formel (I) unten: 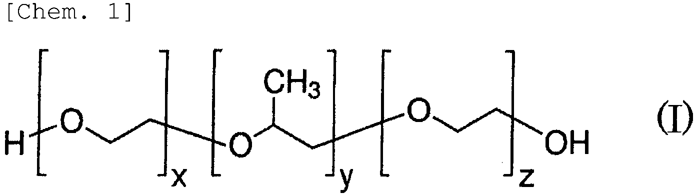 (wobei x, y und z identisch sind oder sich voneinander unterscheiden und jeweils eine ganze Zahl nicht kleiner als 1 sind und
ein Molekulargewicht eines Polypropylenoxidblocks nicht größer als 2750 ist), oder
eine Verbindung, dargestellt durch Formel (II) unten: 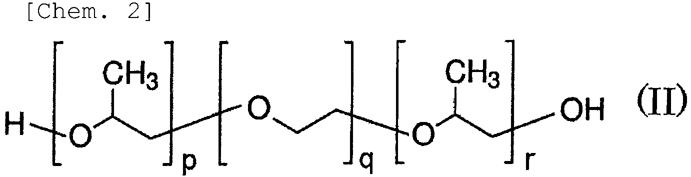 (wobei p, q und r identisch sind oder sich voneinander unterscheiden und jeweils eine ganze Zahl nicht kleiner als 1 sind und
ein Molekulargewicht einer Polypropylenoxideinheit nicht größer als 2750 ist).

5. Verfahren gemäß irgendeinem der Ansprüche 1 bis 4, wobei
das markierte Sterin ein getaggtes Cholesterin ist, dargestellt durch Formel (III) unten: 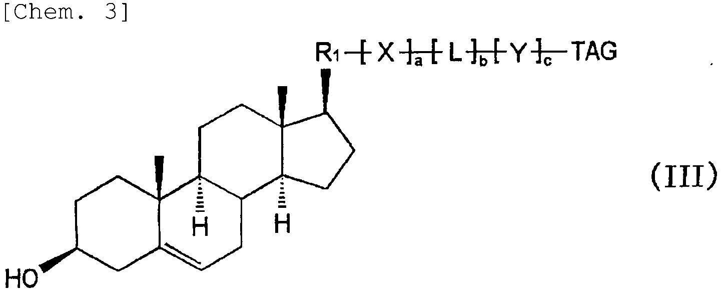 (wobei R₁ eine Alkylengruppe mit 1 bis 6 Kohlenstoffatomen ist und die optional eine Methylgruppe aufweist,
X und Y identisch sind oder sich voneinander unterscheiden und jeweils dargestellt sind durch -R₂-NH-, -NH-R₂-, -R₂- (C=O) -NH-, -(C=O) -NH-R₂-, -R₂-NH-(C=O)-, -NH-(C=O)-R₂-, -R₂-(C=O)-, -(C=O)-R₂-, -R₂-(C=O)-O-, -(C=O)-O-R₂-, -R₂-O-(C=O)-, -O-(C=O)-R₂-, -R₂-(C=S)-NH-, -(C=S)-NH-R₂-, -R₂-NH-(C=S)-, -NH-(C=S)-R₂-, -R₂-O-, -O-R₂-, -R₂-S- oder -S-R₂- und jeder R₂ unabhängig ist: eine atomare Bindung; eine Alkylengruppe mit 1 bis 10 Kohlenstoffatomen und die optional einen Substituenten aufweist; eine Arylengruppe oder Heteroarylengruppe mit 6 bis 12 Kohlenstoffatomen und die optional einen Substituenten aufweist; oder eine Cycloalkylengruppe oder Heterocycloalkylengruppe mit 3 bis 8 Kohlenstoffatomen und die optional einen Substituenten aufweist,
L dargestellt ist durch -(CH₂)_{d}-[R₃-(CH₂)ₑ]_{f}- oder -[(CH₂)ₑ-R₃]_{f}-(CH₂)_{d}- und R₃ ein Sauerstoffatom, ein Schwefelatom, -NH-, -NH-(C=O)- oder -(C=O)-NH- ist,
TAG ein Tag ist,
a und c identisch sind oder sich voneinander unterscheiden und jeweils eine ganze Zahl von 0 bis 6 sind,
b 0 oder 1 ist,
d und e identisch sind oder sich voneinander unterscheiden und jeweils eine ganze Zahl von 0 bis 12 sind und
f eine ganze Zahl von 0 bis 24 ist.).

6. Verfahren gemäß Anspruch 5, wobei
das getaggte Cholesterin als Tag eine Struktur umfasst, der hinzugefügt ist:
- eine Struktur, dargestellt durch Formel (IV) unten: 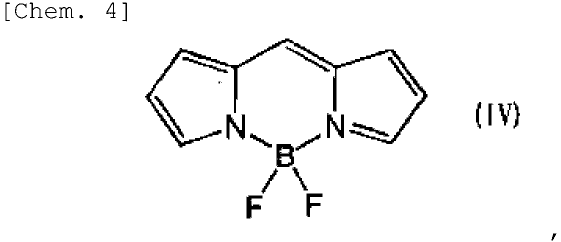
- eine Struktur, dargestellt durch Formel (V) unten: 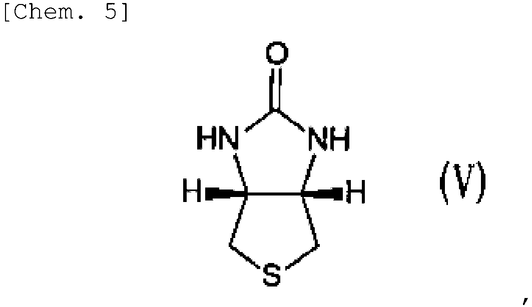 oder
- eine 2,4-Dinitrophenylgruppe.

7. Verfahren gemäß Anspruch 6, wobei
das getaggte Cholesterin dargestellt ist durch Formel (VI) unten: 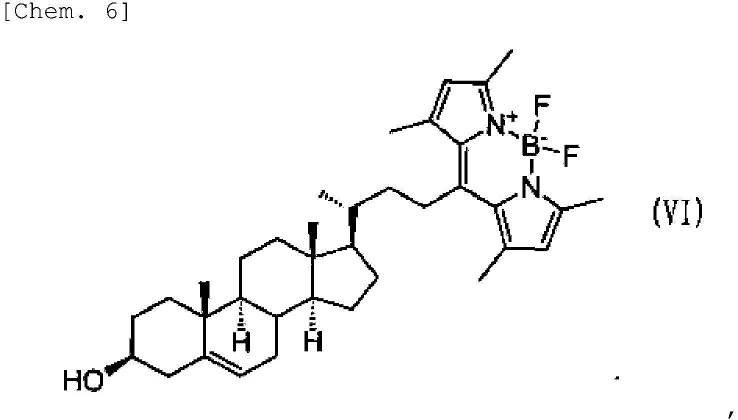
oder Formel (VII) unten: 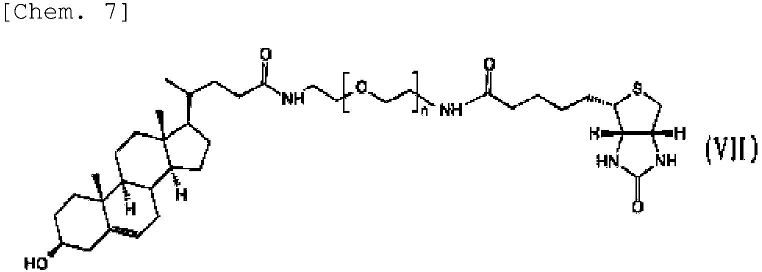 (wobei n eine ganze Zahl von 0 bis 24 ist.),
oder Formel (VIII) unten:

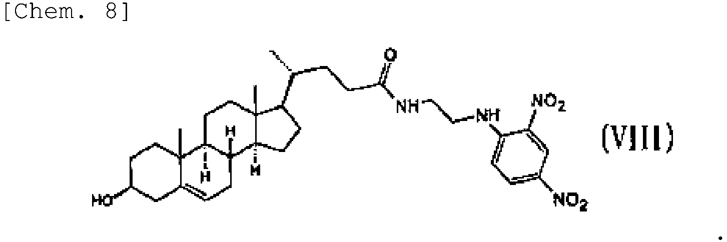

8. Verfahren gemäß irgendeinem der Ansprüche 1 bis 7, wobei
das Messen das Mischen des Lipoproteins, welches das markierte Sterin inkorporiert, und eines ersten Einfangkörpers, der an das Lipoprotein bindet, unter Bildung eines Komplexes, der das Lipoprotein, welches das markierte Sterin inkorporiert, und den ersten Einfangkörper enthält, umfasst.

9. Verfahren gemäß irgendeinem der Ansprüche 1 bis 8, wobei
das Erzeugen in Gegenwart eines 2-Methacryloyloxyethylphosphorylcholin-basierten Polymers durchgeführt wird.

10. Verfahren gemäß irgendeinem der Ansprüche 1 bis 9, wobei die Probe eine Probe ist, die vor dem Erzeugen mit einem wässrigen Medium verdünnt wird, das ein Tensid enthält, welches keine zyklische Struktur aufweist.

11. Verfahren gemäß Anspruch 10, wobei
das Verdünnen in Gegenwart eines zyklischen Oligosaccharids und/oder einer Komponente, die an ein Lipoprotein bindet, welches sich von einem zu messenden Lipoprotein unterscheidet, durchgeführt wird.

12. Verfahren gemäß Anspruch 11, wobei
das zyklische Oligosaccharid Cyclodextrin, Methyl-β-cyclodextrin oder Hydroxypropylcyclodextrin ist und die Komponente, die an das Lipoprotein bindet, welches sich von dem zu messenden Lipoprotein unterscheidet, Calixaren ist.

13. Verfahren gemäß irgendeinem der Ansprüche 1 bis 12, ferner umfassend
Waschen zwischen dem Erzeugen und dem Messen, wobei eine nicht umgesetzte freie Komponente in Gegenwart eines Tensids, welches keine zyklische Struktur aufweist, entfernt wird.

14. Verwendung eines Tensids, welches keine zyklische Struktur aufweist, für das Verfahren gemäß irgendeinem der Ansprüche 1 bis 13.

## Revendications

1. Procédé de mesure d'une capacité d'apport en une lipoprotéine, le procédé comprenant les étapes consistant à :
préparer une lipoprotéine incorporant un stérol marqué, en mélangeant la lipoprotéine dans un échantillon et le stérol marqué en présence d'un tensioactif ne présentant pas de structure cyclique ; et
mesurer une capacité d'apport en le stérol marqué sur la base d'un marquage du stérol marqué incorporé dans la lipoprotéine,
dans lequel le tensioactif ne présentant pas de structure cyclique est un adduit d'oxyde d'éthylène de polyalkylène glycol et/ou un éther de lauryle de polyoxyéthylène.

2. Procédé selon la revendication 1, dans lequel le tensioactif est un tensioactif non ionique.

3. Procédé selon la revendication 1, dans lequel l'adduit d'oxyde d'éthylène de polyalkylène glycol est un d'un type à copolymérisation en blocs.

4. Procédé selon la revendication 3, dans lequel
le tensioactif du type à copolymérisation en blocs est un composé représenté par la formule (I) ci-dessous : 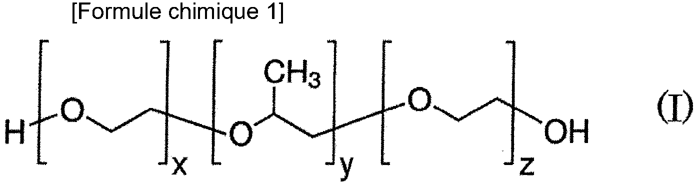 (où x, y et z sont identiques ou différents les uns des autres, et sont chacun un entier non inférieur à 1, et
un poids moléculaire d'un bloc d'oxyde de polypropylène n'est pas supérieur à 2 750), ou
un composé représenté par la formule (II) ci-dessous : 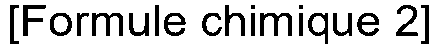 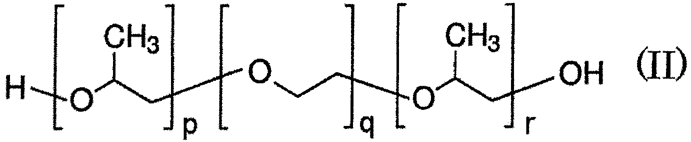 (où p, q et r sont identiques ou différents les uns des autres, et sont chacun un entier non inférieur à 1, et
un poids moléculaire d'une fraction d'oxyde de polypropylène n'est pas supérieur à 2 750).

5. Procédé selon l'une quelconque des revendications 1 à 4, dans lequel
le stérol marqué est un cholestérol étiqueté représenté par la formule (III) ci-dessous : 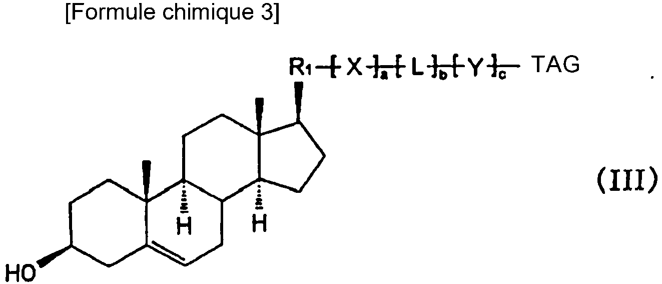 (où R₁ est un groupe alkylène présentant 1 à 6 atomes de carbone et présentant facultativement un groupe méthyle,
X et Y sont identiques ou différents l'un de l'autre, et sont chacun représentés par - R₂-NH-, -NH-R₂-, -R₂-(C=O)-NH-, -(C=O)-NH-R₂-, -R₂-NH-(C=O)-, -NH-(C=O)-R₂-, -R₂-(C=O)-, -(C=O)-R₂-, -R₂-(C=O)-O-, -(C=O)-O-R₂-, -R₂-O-(C=O)-, -O-(C=O)-R₂-, -R₂-(C=S)-NH-, -(C=S)-NH-R₂-, -R₂-NH-(C=S)-, -NH-(C=S)-R₂-, -R₂-O-, -O-R₂-, -R₂-S-, ou -S-R₂-, et chaque R₂ est indépendamment : une liaison atomique ; un groupe alkylène présentant 1 à 10 atomes de carbone et présentant facultativement un substituant ; un groupe arylène ou groupe hétéroarylène présentant 6 à 12 atomes de carbone et présentant facultativement un substituant ; ou un groupe cycloalkylène ou groupe hétérocycloalkylène présentant 3 à 8 atomes de carbone et présentant facultativement un substituant,
L est représenté par -(CH₂)_{d}-[R₃-(CH₂)e]r- ou -[(CH₂)ₑ-R₃]_{f}-(CH₂)_{d}-, et R₃ est un atome d'oxygène, un atome de soufre, -NH-, -NH-(C=O)-, ou -(C=O)-NH-,
TAG est une étiquette,
a et c sont identiques ou différents l'un de l'autre, et sont chacun un entier de 0 à 6,
b est 0 ou 1,
d et e sont identiques ou différents l'un de l'autre, et sont chacun un entier de 0 à 12, et
f est un entier de 0 à 24.)

6. Procédé selon la revendication 5, dans lequel
le cholestérol étiqueté comprend, en tant qu'étiquette, une structure ajoutée avec
- une structure représentée par la formule (IV) ci-dessous : 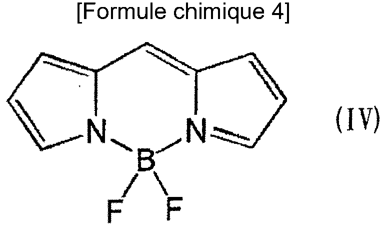
- une structure représentée par la formule (V) ci-dessous : 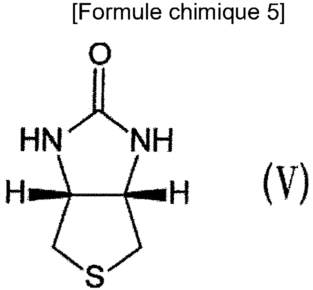 ou
- un groupe 2,4-dinitrophényle.

7. Procédé selon la revendication 6, dans lequel
le cholestérol étiqueté est représenté par la formule (VI) ci-dessous : 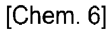 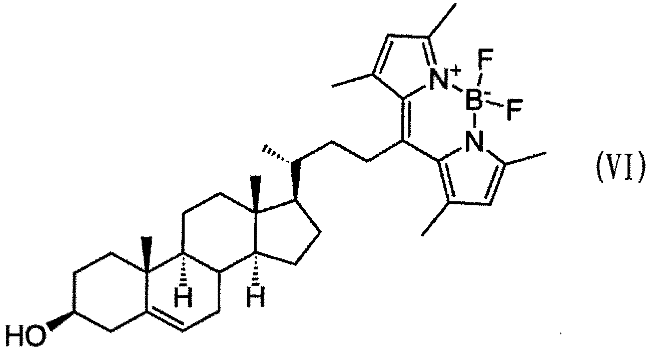
ou la formule (VII) ci-dessous : 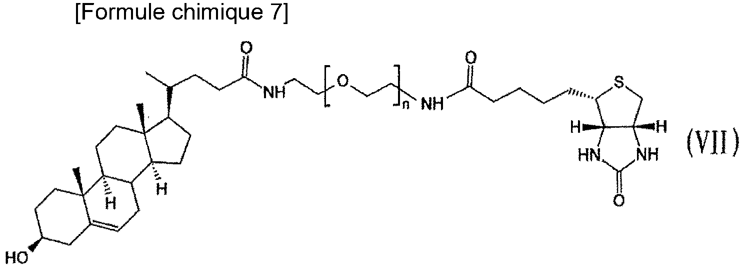 (où n est un entier de 0 à 24.),
ou la formule (VIII) ci-dessous :

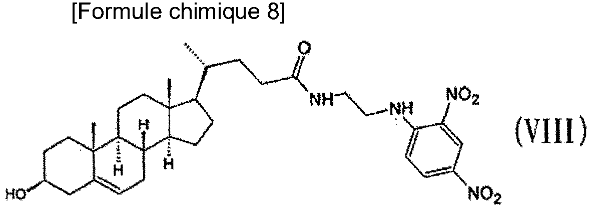

8. Procédé selon l'une quelconque des revendications 1 à 7, dans lequel
la mesure comprend le mélange de la lipoprotéine incorporant le stérol marqué et d'un premier corps de capture qui se lie à la lipoprotéine, pour former un complexe qui contient la lipoprotéine incorporant le stérol marqué et le premier corps de capture.

9. Procédé selon l'une quelconque des revendications 1 à 8, dans lequel
la préparation est réalisée en présence d'un polymère à base de 2-(méthacryloyloxyéthyl)phosphorylcholine.

10. Procédé selon l'une quelconque des revendications 1 à 9, dans lequel
l'échantillon est un échantillon dilué, avant la préparation, avec un milieu aqueux contenant un tensioactif ne présentant pas de structure cyclique.

11. Procédé selon la revendication 10, dans lequel
la dilution est réalisée en présence d'un oligosaccharide cyclique et/ou d'un composant qui se lie à une lipoprotéine différente d'une lipoprotéine devant être mesurée.

12. Procédé selon la revendication 11, dans lequel
l'oligosaccharide cyclique est une cyclodextrine, une méthyl-β-cyclodextrine, ou une hydroxypropyl-cyclodextrine, et le composant qui se lie à la lipoprotéine différente de la lipoprotéine devant être mesurée est un calixarène.

13. Procédé selon l'une quelconque des revendications 1 à 12, comprenant en outre
entre la préparation et la mesure, un lavage dans lequel un composant libre non réagi est supprimé en présence d'un tensioactif ne présentant pas de structure cyclique.

14. Utilisation d'un tensioactif ne présentant pas de structure cyclique pour le procédé selon l'une quelconque des revendications 1 à 13.
